# EUROPÄISCHE PATENTSCHRIFT

(11) **EP 0 850 492 B1**
(45) Veröffentlichungstag und Bekanntmachung des Hinweises auf die Patenterteilung: **12.03.2003**
(21) Anmeldenummer: 96931054.9
(22) Anmeldetag: 09.09.1996
(51) Int. Cl.: H01L 51/30

(54) **PHOTOVOLTAISCHE ZELLE MIT EINER SPIROVERBINDUNG**
PHOTO-VOLTAIC CELL WITH A SPIRO-COMPOUND
CELLULE PHOTOVOLTAIQUE AVEC UN COMPOSE SPIRANNIQUE

(30) Priorität: 13.09.1995 DE 19533850
(43) Veröffentlichungstag der Anmeldung: 01.07.1998
(73) Patentinhaber: Covion Organic Semiconductors GmbH, 65926 Frankfurt (DE)
(72) Erfinder: LUPO, Donald, D-60316 Frankfurt am Main (DE); SALBECK, Josef, D-65779 Kelkheim (DE)
(74) Vertreter: Luderschmidt, Schüler & Partner GbR
(86) Internationale Anmeldenummer: EP9603944
(87) Internationale Veröffentlichungsnummer: WO97010617

(56) Entgegenhaltungen:
- EP-A- 0 333 641
- EP-A- 0 452 078
- US-A- 5 026 894
- DATABASE WPI Section Ch, Week 9423 Derwent Publications Ltd., London, GB; Class A12, AN 94-186208 XP002022370 & JP 06 122 277 (TOSHIBA KK) , 6.Mai 1994 & US 5 536 588 A (KATSUYUKI NAITO) 16.Juli 1996

## Beschreibung

Aufgrund des weltweit steigenden Bedarfs an elektrischer Energie und der begrenzten Vorräte an Kohle, Öl und Gas, die bei ihrer Verbrennung zudem das Treibhausgas CO₂ freisetzen, hat die Erzeugung von elektronischem Strom aus Sonnenlicht in den letzten Jahre erhöhtes Interesse gefunden.

Unter einer photovoltaischen Zelle versteht man ein Bauelement, in dem Licht direkt in elektrische Energie umgewandelt wird. Es enhält neben zwei Elektroden mindestens eine lichtabsorbierende Schicht und eine Ladungstransportschicht.
Handelt es sich um Sonnenlicht, spricht man von einer Solarzelle.
Obwohl der photovoltaische Effekt, d. h. das Auftreten einer Spannung über einem pn-Übergang bei Einstrahlung von Licht, bereits 1893 von Becquerel beobachtet wurde, gelang es erst mit der Entwicklung der Halbleitertechnik in den vierziger und fünfziger Jahren dieses Jahrhunderts, die ersten funktionsfähigen Solarzellen herzustellen.
Die heute verwendeten Solarzellen enthalten üblicherweise als lichtabsorbierende Schicht ein Halbleitermaterial, in den meisten Fällen Silicium, das für diese Anwendung allerdings höchsten Ansprüchen an die Reinheit und Qualität der Verarbeitung genügen muß. Dadurch sind Solarzellen heute bei vielen Anwendungen aus Kostengründen nicht konkurrenzfähig.
Demgegenüber wird in einer Farbstoff-sensibilisierten Solarzelle, wie sie in den sechziger Jahren von H. Tributsch entwickelt wurde, als Halbleiter ein Material mit sehr großer Bandlücke, wie Titandioxid, verwendet. Ein solcher Halbleiter absorbiert das Sonnenlicht nur wenig, hierfür wird auf den Halbleiter ein Farbstoff (Chromophor) aufgetragen.

Wird in einem Farbstoffmolekül ein Photon absorbiert, so bewirkt dieses die Anregung eines Elektrons in den niedrigsten unbesetzten Molekülzustand. Aus diesem kann es dann in das Leitungsband des Halbleiters injiziert werden. Der Halbleiter dient also überwiegend dem Transport von Elektronen. Hierfür ist keine besondere Reinheit und Perfektion des Materials notwendig. Es kann z. B. aus einer Pulversuspension einfach auf leitfähiges Glas gestrichen werden.

In der EP-A O 333 641 ist eine photoelektrochemische Zelle beschrieben, die dadurch gekennzeichnet ist, daß sie einen nanoporösen, d. h. eine extrem aufgerauhte und damit vergrößerte Oberfläche aufweisenden Metalloxid-Halbleiter enthält. Der Ladungstransport zwischen Halbleiter/Chromophor-Schicht und Gegenelektrode erfolgt in dieser Zelle durch eine Elektrolytlösung.
Obwohl mit solchen Zellen gute Ergebnisse erzielt werden, ist das Eigenschaftsprofil einer solchen Vorrichtung noch deutlich verbesserungsfähig.
Insbesondere nachteilig ist, daß das Elektron durch die Diffusion von Ionen an den Farbstoff zurückgeführt werden muß. Dadurch kommen nur Redoxsysteme in Frage, die klein genug sind, um in die Poren der nanokristallinen Halbleiterschicht einzudringen. Selbst bei dem bisher besten Redox-System, I₂/I₃ geht ungefähr 40 % der theoretisch zur Verfügung stehenden Energie als Wärme verloren, und der Wirkungsgrad für die Energiekonversion ist auf ca. 10 % im Sonnenlicht begrenzt. Zudem muß im Elektrolyt stets ein Kompromiß zwischen Viskosität und Mobilität der Ionen gefunden werden, der keiner der beiden Anforderungen ideal genügt.

Es wurde nun überraschend gefunden, daß sich die oben beschriebenen Nachteile zumindest verringern lassen, wenn die Elektrolytschicht in der oben diskutierten Zelle durch eine Ladungstransportschicht aus einem Lochleitermaterial ersetzt wird.

Gegenstand der Erfindung ist daher eine photovoltaische Zelle enthaltend eine Ladungstransportschicht, die ein oder mehrere Spiro- und Heterospiroverbindungen der Formel (I) 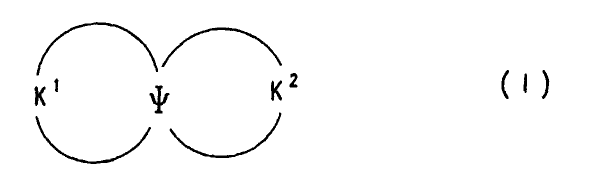 wobei
Ψ C, Si, Ge oder Sn, vorzugsweise C, Si oder Ge, besonders bevorzugt C oder
Si und insbesondere C und
K¹ und K² unabhängig voneinander konjugierte Systeme
bedeuten, als Lochleitermaterialien enthält.

Durch die Verwendung des Lochleitermaterials anstelle des Elektrolyten ist der Landungstransport nicht mehr durch die Diffusion der lonen begrenzt. Zusätzlich können die relevanten Energieniveaus der Schicht so angepaßt werden, daß der Wirkungsgrad für Sonnenlicht auf ≥ 18 % verbessert werden kann.

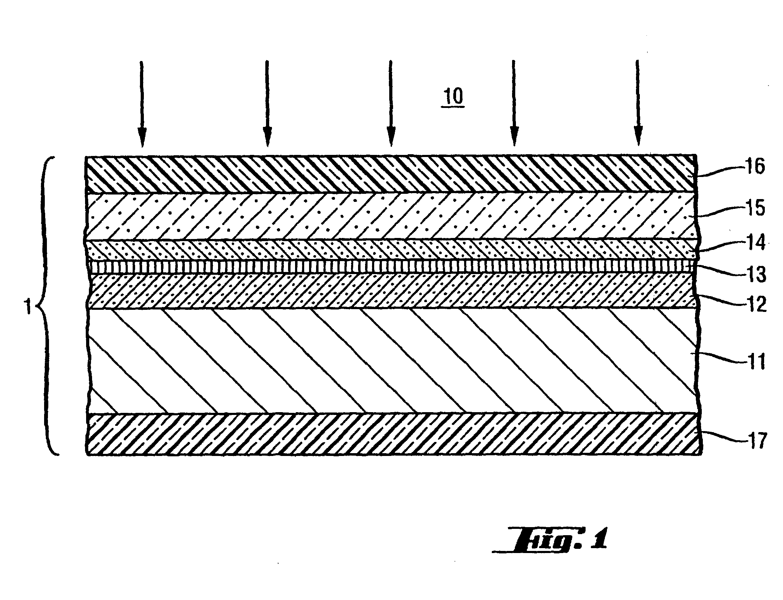

In Fig. 1 ist eine bevorzugte Ausführungsform der erfindungsgemäßen Zelle 1 dargestellt (nicht maßstabsgetreu).
Auf einem leitenden Träger 11, der als Elektrode bzw. Kontakt dienen kann und beispielsweise aus einem Metall oder Indium-Zinn-Oxid (ITO) besteht, ist als lichtabsorbierende Schicht ein Halbleiter 12 aufgebracht, der vorzugsweise eine Oberfläche mit einem Rauheitsfaktor > 1 hat. Vorzugsweise enthält die erfindungsgemäße Zelle auf der Oberfläche des Halbleiters einen Chromophor, hier als Chromophorschicht 13 dargestellt. Die Bezeichnung lichtabsorbierende Schicht umfaßt im Sinne der Erfindung sowohl eine Halbleiterschicht als auch eine Kombination Halbleiter/Chromophor, auch wenn die eigentliche Absorption in diesem Fall nahezu ausschließlich durch den Chromophor erfolgt. Daran schließt sich die Ladungstransportschicht 14 an, die erfindungsgemäß ein Lochleitermaterial der Formel (I) enthält. Sie wird auf der einen Seite durch die Gegenelektrode 15 begrenzt, die beispielsweise aus einem leitfähigen Glas, leitfähig beschichtetem Kunststoff, aus Metall oder einem anderen leitfähigen, vorzugsweise lichtdurchlässigen Material, bestehen kann. Die Zelle 1 ist vorzugsweise oben und unten durch je eine isolierende Schicht 16 bzw. 17 abgeschlossen. Sie kann einen, in der Figur nicht gezeigten seitlichen Abschluß enthalten, beispielsweise einen Rahmen aus einem elektrisch isolierendem Material, wie Kunststoff oder Glas. Durch die Verwendung eines Lochleitermaterials anstelle des flüssigen Elektrolyten ist solch ein seitlicher Rahmen aber nicht grundsätzlich notwendig. Zumindest eine Seite der Zelle muß für das in elektrische Energie zu wandelnde Licht durchlässig sein, so daß dieses zu dem Chromophor bzw. dem Halbleiter gelangen kann.
Die erfindungsgemäße Zelle enthält darüber hinaus in der Figur nicht gezeigte Vorrichtungen zur Abnahme des von der Zelle erzeugten elektrischen Stroms.

Als Lochleitermaterial im Sinne der Erfindung gilt ein Material, das eine positive Ladung leiten kann, die durch das Fehlen eines Elektons zustande kommt, wobei Massentransport und Ladungstransport entkoppelt sind.

Allgemein eignen sich elektronenreiche, vorzugsweise organische Verbindungen, die, vorzugsweise reversibel, oxidierbar sind. Es wird allgemein angenommen, daß der Ladungstransport in einem organischen Lochleitermaterial über die Bildung von Radikalkationen erfolgt.

Das Oxidationspotential ist dabei in weiten Bereichen variabel und kann an die spezifischen Energieniveaus des Halbleiters oder Sensibilisators angepaßt werden. Es liegt vorzugsweise zwischen dem Energienieveau des Grundzustands und 700, vorzugsweise 400, besonders bevorzugt 300, mV oberhalb des Grundzustands.

Bevorzugt sind Lochleitermaterialien, bei denen kein Massentransport stattfindet, bzw. bei denen Ladungs- und Massentransport völlig entkoppelt sind.
Weiterhin bevorzugt sind feste, insbesondere amorphe Lochleitermaterialien der Formel (I).

Dabei ist es im Sinne der Erfindung bevorzugt, daß die erfindungsgemäße Lochleiterschicht amorph präparierbar ist, d.h. im amorphen Zustand in der erfindungsgemäßen photovoltarischen Zelle aufgebracht wird.

Amorph dient zur Zustandsbeschreibung von Festkörpern, deren molekulare Bausteine nicht in Kristallgittern, sondern regellos angeordnet sind. Anders als in einem Kristall, bei dem zwischenden Atomen eine Nahordnung (d.h. konstante Abstände zu nächsten Nachbaratomen) und eine Fernordnung (regelmäßiges Wiederholen eines Basisgitters) existiert, liegt im amorphen Zustand nur eine Nahordnung vor. Der amorphe Stoff besitzt keine physikalisch ausgezeichnete Richtung; er ist isotrop. Alle amorphen Stoffe streben unterschiedlich stark den energetisch günstigeren kristallinen Zustand an. Bei Beugung von Röntgen-, Elektronen- und Neutronenstrahlen zeigen sich beim amorphen Festkörper keine scharfen Interferenz-Ringe, wie bei einem Kristall, sondern nur diffuse Interferenzringe bei kleinen Beugungswinkeln (Halos).

Der amorphe Zustand ist somit klar vom kristallinen, flüssigen oder auch flüssigkristallinen Zustand unterschieden.

Besonders bevorzugt sind Lochleitermaterialien, die in organischen Lösungsmitteln löslich sind, sowie Lochleitermaterialien, die schmelzbar sind. Beispiele für organische Lösungsmittel, auf die aber die Erfindung nicht eingeschränkt ist, sind Chloroform, Benzol, Chlorbenzol, Cyclohexanon, Toluol, Tetrahydrofuran, Anisol, Cresol, Xylol, Methyllaktat, Methylenchlorid, Hexan, oder andere aliphatische, aromatische oder alkoholische Lösungsmittel. Es reicht für die Herstellung einer erfindungsgemäßen Lochleiterschicht, wenn das Lochleitermaterial in einem organischen Lösungsmittel löslich ist oder schmelzbar ist. Löslich im Sinne der Erfindung bedeutet dabei eine Löslichkeit von mindestens 1,0 g/l bei 25°C in einem organischen oder anorganischen Lösungsmitel, vorzugsweise in einem der oben aufgeführten Lösungsmittel.

Weiterhin besonders bevorzugt sind Lochleitermaterialien, die anhand ihrer Größe in die Poren einer rauhen Halbleiterschicht eindiffundieren können.

Dazu ist es bevorzugt, daß die Moleküle der Lochtransportschicht, eine vergleichbare molekulare Dimension wie die, gegebenenfalls verwendeten, Moleküle des Sensibilisators aufweisen, damit die Lochleitermoleküle in engen Kontakt mit den in den Poren der Halbleiteroberfläche sitzenden Sensibilisatormolekülen kommen können. Besonders bevorzugt sind die Lochleitermoleküle weniger als den Faktor 20, ganz besonders bevorzugt 10, größer als entsprechende Sensiblisatormoleküle. Ganz besonders bevorzugt sind Lochleitermaterialien, bei denen der Spannungsverlust über die Lochleiterschicht bei Bestrahlung mit Sonnenlicht <500 mV, vorzugsweise < 50 mV, besonders bevorzugt < 20 mV beträgt.
Die Lochleiterschicht weist im allgemeinen eine Dicke von 0,1 bis 20, vorzugsweise 1 bis 15 µm auf.
Die Ladungsträgerbeweglichkeit des Lochleitermaterials beträgt vorzugsweise mindetens 10⁻⁵ cm²/Vs, insbesondere 10⁻³ bis 10 cm²/Vs.

Spiroverbindungen sind Verbindungen in denen zwei Ringsysteme durch ein einziges, vierbindiges Atom verknüpft sind. Dieses Atom wird als Spiroatom bezeichnet, wie in Handbook of Chemistry and Physics 62^{nd} ed. (1981-2), CRC Press, S. C-23 bis C-25 ausgeführt ist. Der Begriff Spiroverbindung bedeutet im Sinne der Erfindung monomere und polymere Carbospiro- und Heterospiroverbindungen.

Bevorzugte Verbindungen der Formel (I) sind 9,9'-Spirobifluorenderivate der Formel (II), 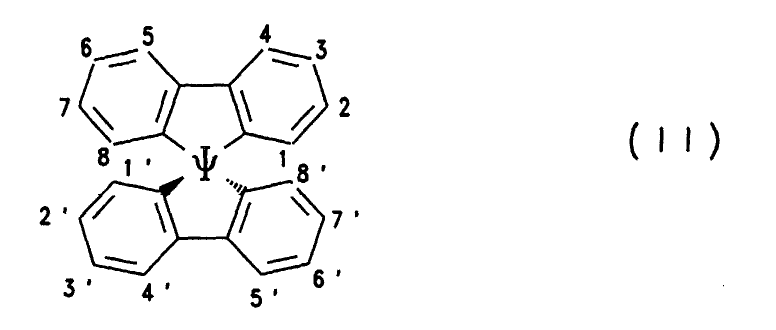 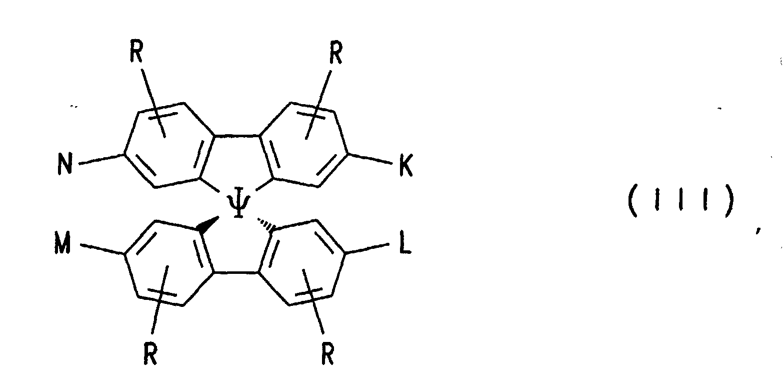 wobei die Symbole und Indizes folgende Bedeutungen haben:
ψ ist C, Si, Ge oder Sn, vorzugsweise C, Si, Ge, besonders bevorzugt C, Si, insbesondere C,
K, L, M, N sind gleich oder verschieden und bedeuten eine Gruppe der Formeln

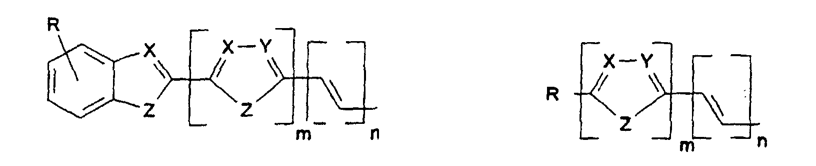

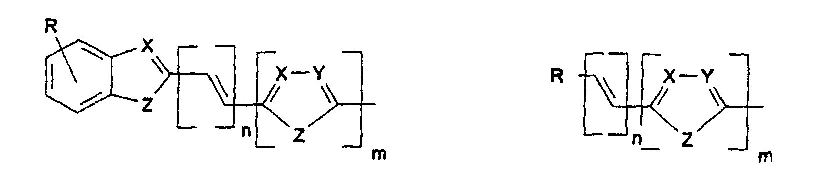

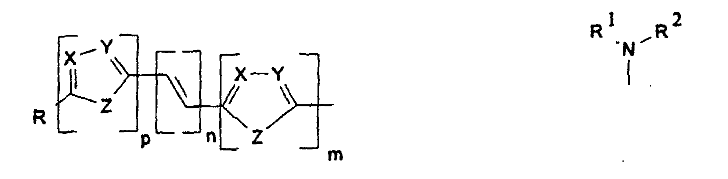

- R: hat, gleich oder verschieden, die gleichen Bedeutungen wie K, L, M, N oder ist Wasserstoff, eine lineare oder verzweigte Alkyl-, Alkoxy- oder Carboalkoxygruppe mit 1 bis 22, vorzugsweise 1 bis 15, besonders bevorzugt 1 bis 12 C-Atomen, -CN, -NO₂, -NR¹R², -Ar oder -O-Ar;
- Ar: ist Phenyl, Biphenyl, 1-Naphthyl, 2-Naphthyl, 2-Thienyl, 2-Furanyl, wobei jede dieser Gruppen einen oder zwei Reste R tragen kann;
- m, n, p: sind 0, 1, 2 oder 3;
- X, Y: sind = CR- oder = N-;
- Z: ist -O-, -S-, -NR-, -CRR-, -CH = CH-, -CH = N-;
- R¹ und R²: sind Wasserstoff, eine lineare oder verzweigte Alkylgruppe mit 1 bis 22 C-Atomen, -Ar oder 3-Methylphenyl.

### Bevorzugte Verbindungen der Formel (III) sind solche der Formel (IIIa)-(IIIg)

IIIa) K = L = M = N und ist eine Gruppe der Formeln: 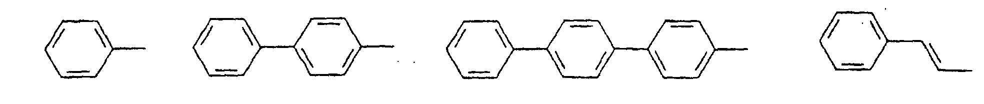 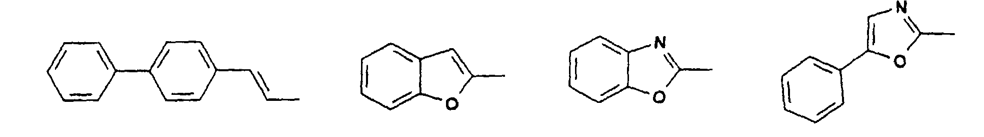 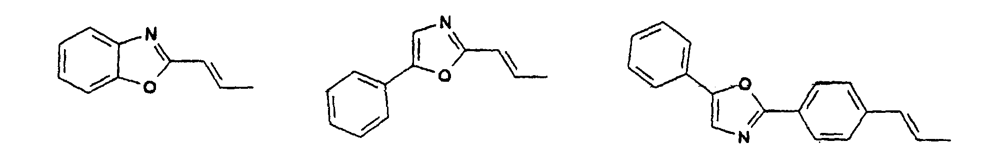 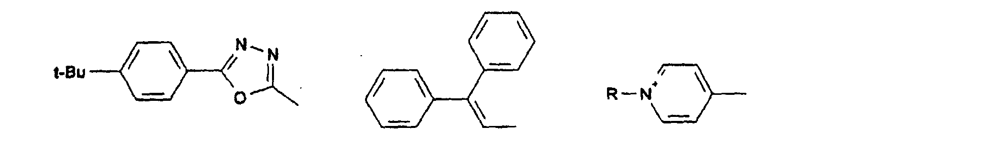 R = C₁-C₂₂-Alkyl, C₂H₄SO₃⁻
IIIb) K = M = H und N = L und ist eine Gruppe der Formeln: 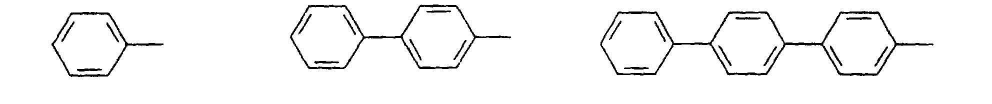 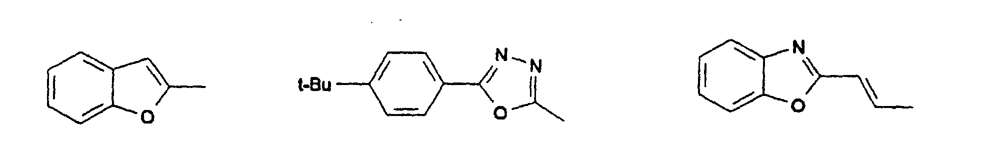 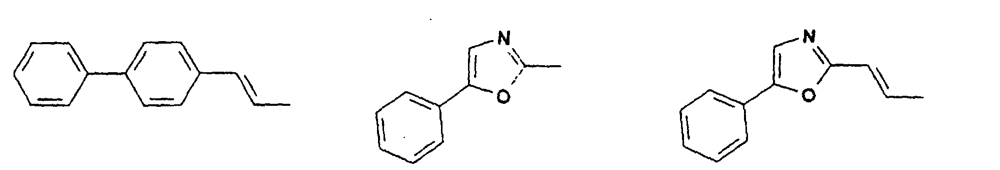 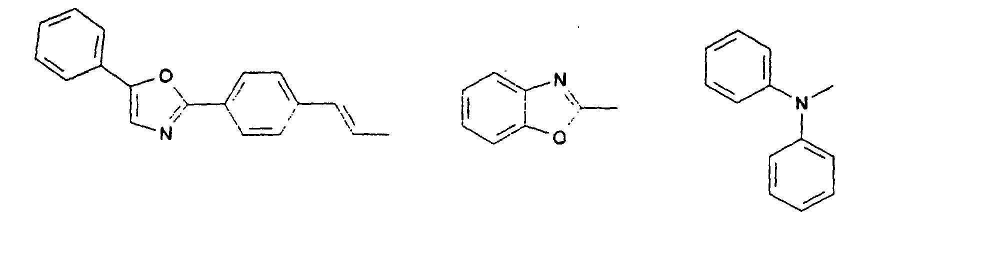
IIIc) K = M und ist eine Gruppe der Formeln: 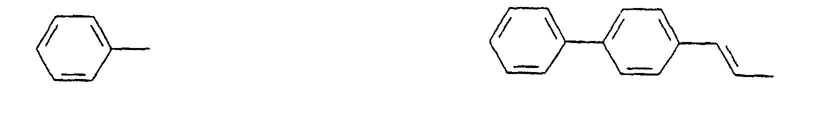 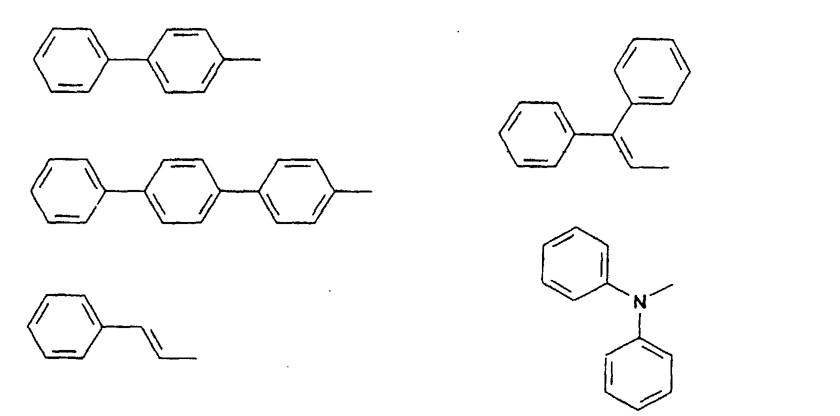 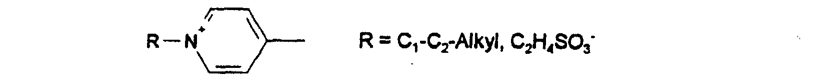 und N = L und ist eine Gruppe der Formeln: 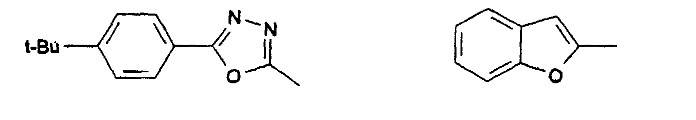 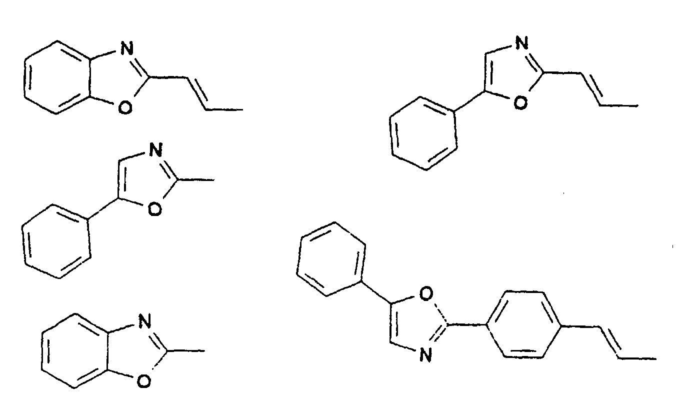
IIId) K = M und ist eine Gruppe der Formeln: 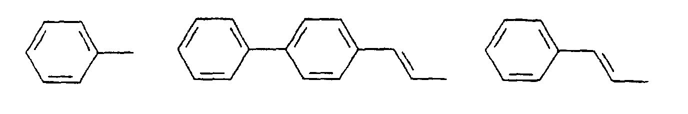 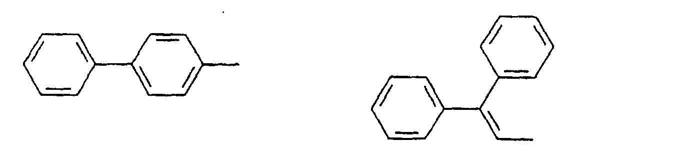 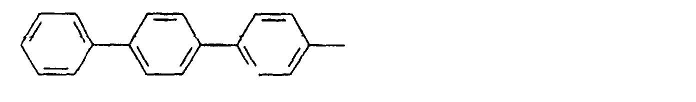 und N = L und ist eine Gruppe der Formeln: 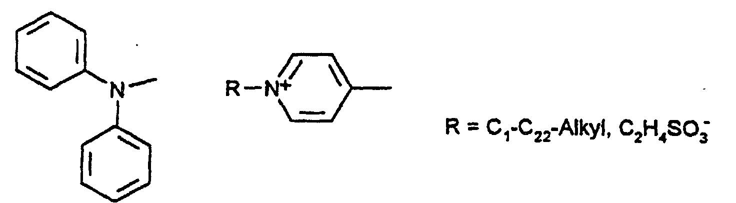
IIIe) K = L = H und M = N und ist eine Gruppe der Formeln: 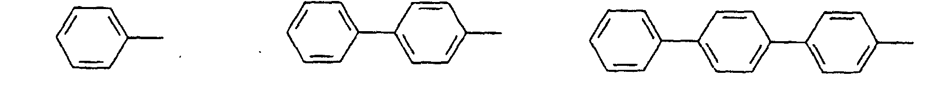 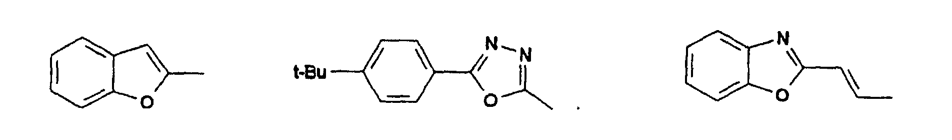 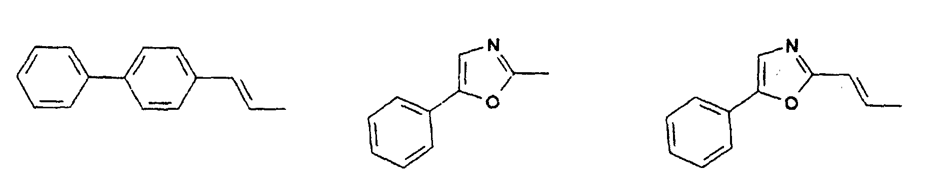 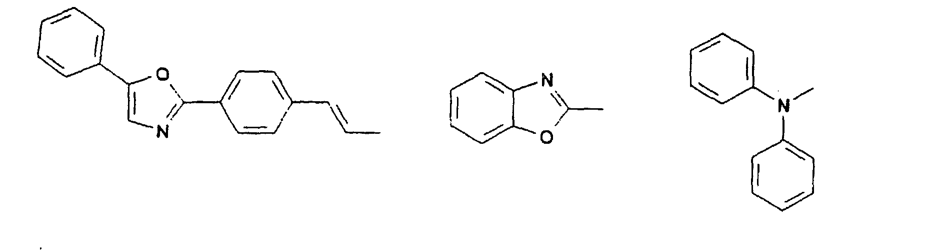
IIIf) K = L und ist eine Gruppe der Formeln: 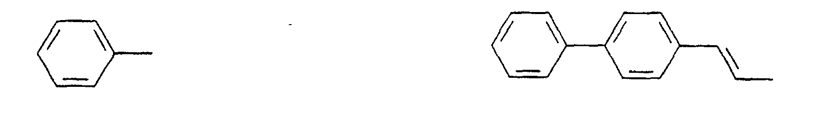 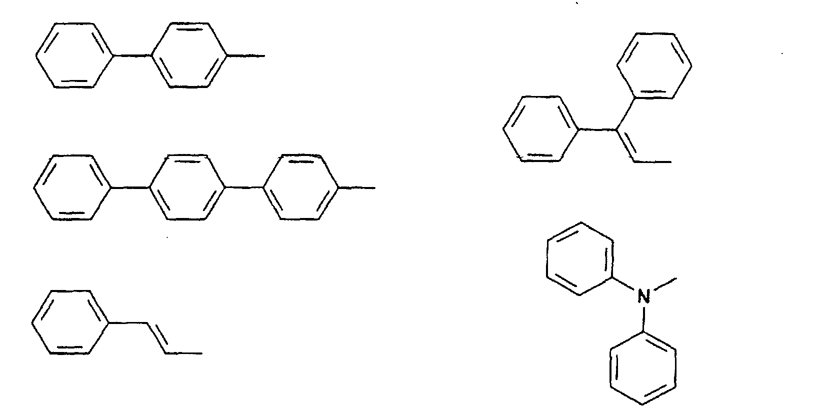 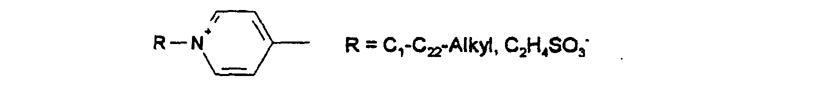 und M = N und ist eine Gruppe der Formeln: 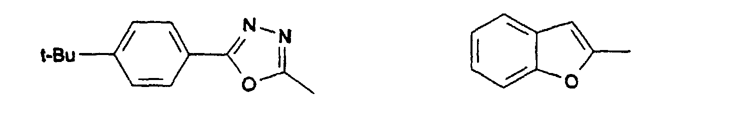 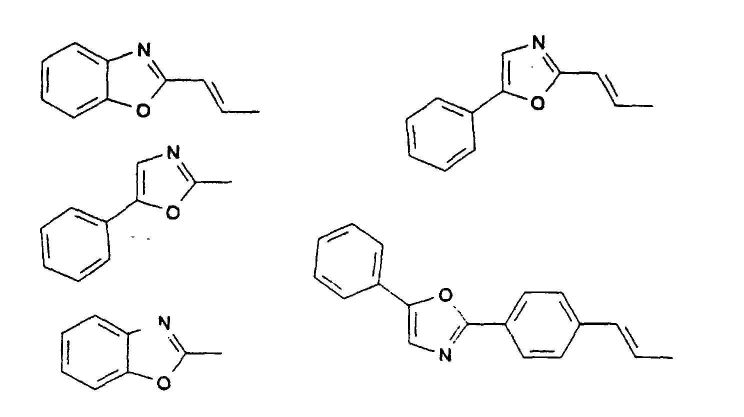
IIIg) K = L und ist aus der Gruppe: 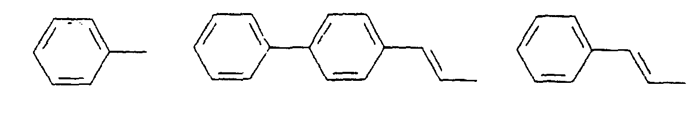 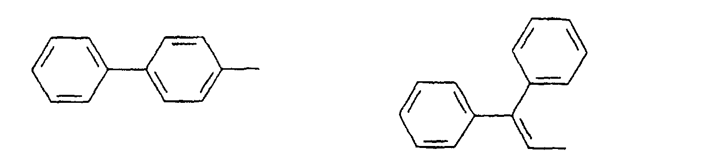 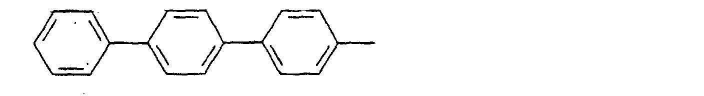 und M =N und ist eine Gruppe der Formeln:

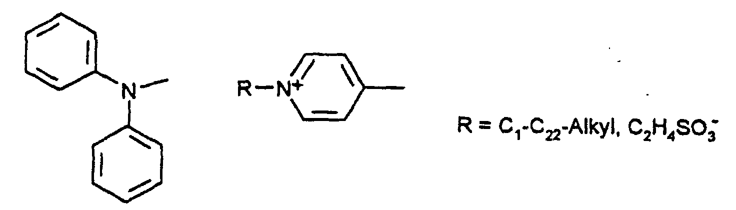

Besonders bevorzugte Verbindungen der Formel (III) sind solche der Formeln (IIIaa) bis (IIIdb):
(IIIaa) K = L = M = N und ist eine Gruppe der Formeln: 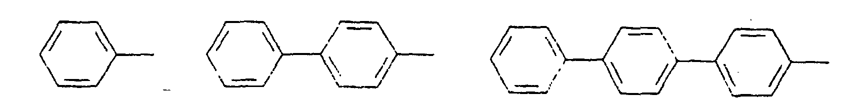
(IIIba) K = M = H und N = L und ist eine Gruppe der Formeln: 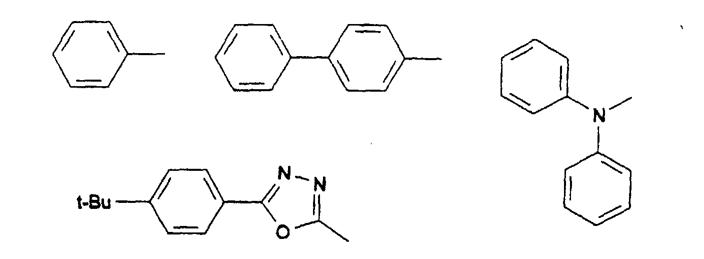
(IIIca) K = M und ist eine Gruppe der Formeln: 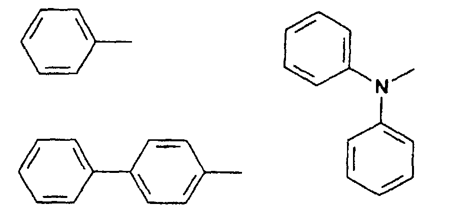 und N = L und ist: 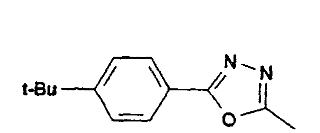
(IIIda) K = M und ist eine Gruppe der Formeln: 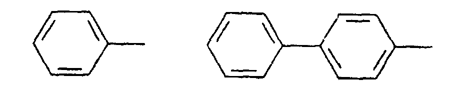 und N = L und ist: 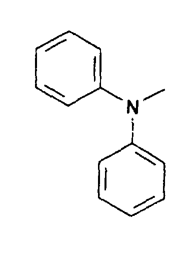
(IIIab) K = L = M = N und ist eine Gruppe der Formeln: 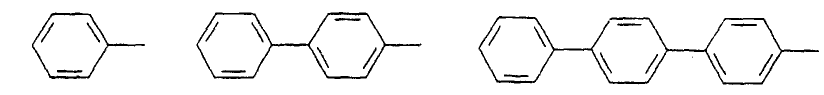
(IIIbb) K = L = H und M = N und ist eine Gruppe der Formeln: 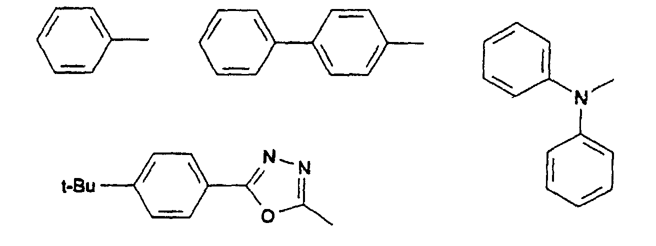
(IIIcb) K = L und ist eine Gruppe der Formeln: 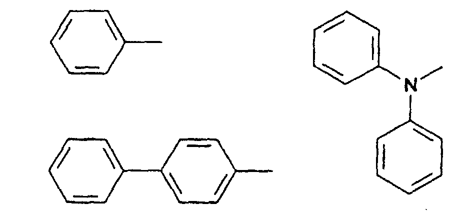 und M = N und ist: 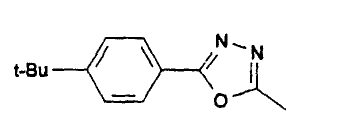
(IIIdb) K = L und ist eine Gruppe der Formeln: 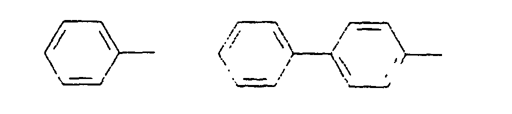 und M = N und ist:

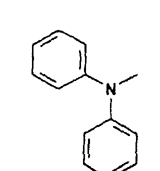

Ganz besonders bevorzugte Spiroverbindungen sind solche der Formel (IV), 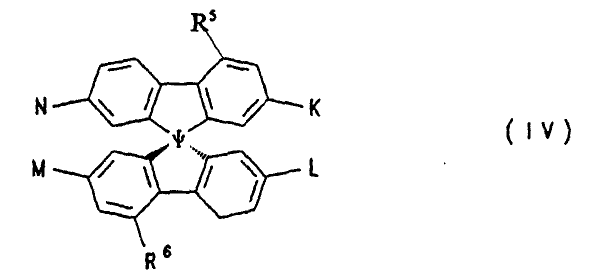 wobei die Symbole folgende Bedeutungen haben:
ψ ist C oder Si, vorzugsweise C;
K, L, M und N sind gleich oder verschieden eine der Gruppen G1 bis G14: 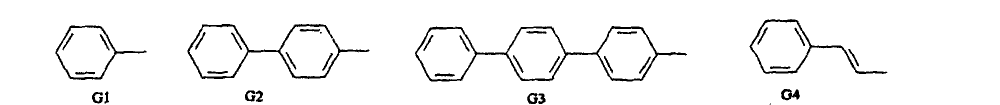 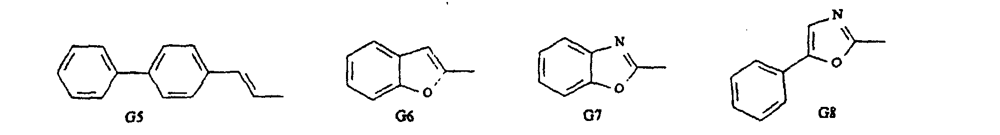 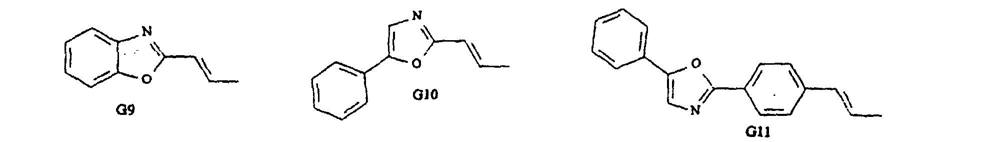 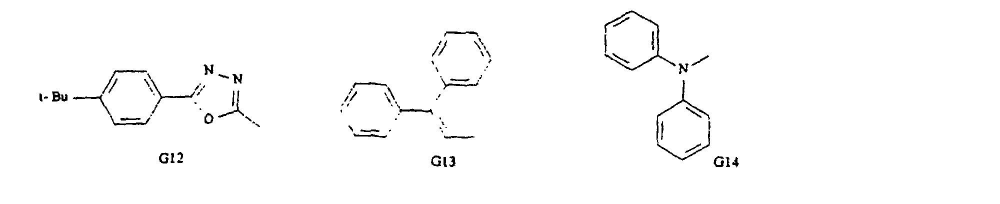
und R⁵, R⁶ können auch gleich oder verschieden Wasserstoff oder eine lineare oder verzweigte Alkyl-, Alkyloxy- oder Estergruppe mit 1 bis 22 C-Atomen, -CN oder -NO₂ bedeuten.

Insbesondere bevorzugte Spiroverbindungen der Formel (IV) sind
2,2',4,4',7,7'-Hexakis(biphenylyl)-9,9'-spirobifluoren,
2,2',4,4',7,7'-Hexakis(terphenylyl)-9,9'-spirobifluoren,
2,2',4,4'-Hexakis(biphenylyl)-9,9'-spirobi-9-silafluoren und
2,2',4,4'7,7'-Hexakis(terphenylyl)-9,9'-spirobi-9-silafluoren.

Die Herstellung der erfindungsgemäß verwendeten Spiro- und Heterospiroverbindungen erfolgt nach an sich bekannten Methoden, wie sie in der EP-A O 676 461 und Standardwerken zur Organischen Synthese, z.B. Houben-Weyl, Methoden der Organischen Chemie, Georg-Thieme-Verlag, Stuttgart und in den entsprechenden Bänden der Serie "The Chemistry of Heterocyclic Compounds" von A. Weissberger und E. C. Taylor (Herausgeber) beschrieben werden.

Die Herstellung erfolgt dabei unter Reaktionsbedingungen, die für die genannten Umsetzungen bekannt und geeignet sind. Dabei kann auch von an sich bekannten, hier nicht näher erwähnten Varianten Gebrauch gemacht werden.

### a) Carbospiroverbindungen

Verbindungen der Formel (III) werden beispielsweise ausgehend vom 9,9'-Spirobifluoren erhalten, dessen Synthese z.B. von R. G. Clarkson, M. Gomberg, J. Am. Chem. Soc. 1030, 52, 2881, beschrieben ist.

Die Herstellung von Verbindungen der Formel (IIIa) kann beispielsweise ausgehend von einer Tetrahalogenierung in den Positionen 2,2',7,7' des 9,9'-Spirobifluorens und anschließender Substitutionsreaktion erfolgen (siehe z.B. US 5,026,894) oder über eine Tetraacetylierung der Positionen 2,2',7,7' des 9,9'-Spirobifluorens mit anschließender C-C-Verknüpfung nach Umwandlung der Acetylgruppen in Aldehydgruppen oder Heterocyclenaufbau nach Umwandlung der Acetylgruppen in Carbonsäuregruppen erfolgen.

Die Herstellung von Verbindungen der Formel (IIIb) kann beispielsweise analog zu denen der Formel IIIa erfolgen, wobei die stöchiometrischen Verhältnisse bei der Umsetzung so gewählt werden, daß die Positionen 2,2' bzw. 7,7' funktionalisiert werden (siehe z.B. J. H. Weisburger, E. K. Weisburger, F. E. Ray, J. Am. Chem. Soc. 1959, 72 4253; F. K. Sutcliffe, H. M. Shahidi, D. Paterson, J. Soc. Dyers Colour 1978, 94, 306 und G. Haas, V. Prelog, Helv. Chim. Acta 1969, 52 1202).

Die Herstellung von Verbindungen der Formel (IIIc) kann beispielsweise über eine Dibromierung in 2,2'Stellung und anschließender Diacetylierung in 7,7' Stellung des 9,9'-Spirobifluorens und anschließende Umsetzung analog zu der der Verbindungen (IIIa) erfolgen.

Verbindungen der Formeln (IIIe)-(IIIg) sind beispielsweise durch Wahl geeignet substituierter Ausgangsverbindungen beim Aufbau des Spirobifluorens herstellbar, z.B. kann 2,7-Dibromspirobifluoren aus 2,7-Dibromfluorenon und 2,7-Dicarbethoxy-9,9'-spirobifluoren durch Einsatz von 2,7-Dicarbethoxyfluorenon aufgebaut werden. Die freien 2',7'-Positionen des Spirobifluorens können dann unabhängig weiter substituiert werden.

Für die Synthese der Gruppen K, L, M, N, sei beispielsweise verwiesen auf DE-A 23 44 732, 24 50 088, 24 29 093, 25 02 904, 26 36 684, 27 01 591
und 27 52 975 für Verbindungen mit 1,4-Phenylen-Gruppen;
DE-A 26 41 724 für Verbindungen mit Pyrimidin-2,5-diyl-Gruppen; DE-A 40 26 223 und EP-A 03 91 203 für Verbindungen mit Pyridin-2,5-diyl-Gruppen;
DE-A 32 31 462 für Verbindungen mit Pyridazin-3,6-diyl-Gruppen; N. Miyaura, T. Yanagi und A. Suzuki in Synthetic Communications 1981, 11, 513 bis 519, DE-A-39 30 663; M. J. Sharp, W. Cheng, V. Snieckus, Tetrahedron Letters 1987, 28, 5093; G. W. Gray, J. Chem. Soc. Perkin Trans II 1989, 2041 und Mol. Cryst. Liq. Cryst. 1989, 172, 165; Mol. Cryst. Liq. Cryst. 1991, 204, 43 und 91; EP-A 0 449 015; WO 89/12039; WO 89/03821; EP-A 0 354 434 für die direkte Verknüpfung von Aromaten und Heteroaromaten.

Die Herstellung disubstituierter Pyridine, disubstituierter Pyrazine, disubstituierter Pyrimidine und disubstituierter Pyridazine findet sich beispielsweise in den entsprechenden Bänden der Serie "The Chemistry of Heterocyclic Compounds" von A. Weissberger und E. C. Taylor (Herausgeber).

### b) Heterospiroverbindungen

Solche Verbindungen der Formel (III) werden beispielsweise ausgehend vom Bis-[biphenyl-2,2'-diyl]silan (=9,9'-Spirobi(9H-)-silafluoren) (V) erhalten, dessen Synthese z.B. von H. Gilman, R. D. Gorsich, J. Am. Chem. Soc. 1958, 80, 3243, beschrieben ist.

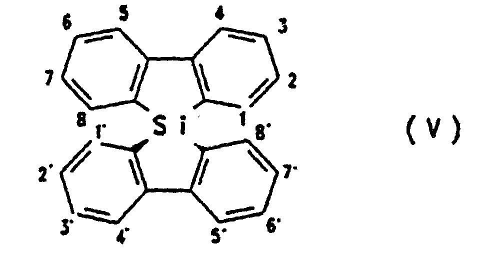

Die Herstellung von Heterospiroverbindungen der Formel (IIIa) kann beispielsweise ausgehend von einer Tetrahalogenierung in den Positionen 2,2',7,7' des 9,9'-Spirobi-9-silafluorens und anschließender Substitutionsreaktion erfolgen, die von analogen C-Spiroverbindungen bekannt (siehe z.B. US 5,026,894) oder über eine Tetraacetylierung der Positionen 2,2',7,7' des 9,9'-Spirobi-9-silafluorens mit anschließender C-C-Verknüpfung nach Umwandlung der Acetylgruppen in Aldehydgruppen oder Heterocyclenaufbau nach Umwandlung der Acetylgruppen in Carbonsäuregruppen erfolgen.
Die Herstellung von Verbindungen der Formel (IIIb) kann beispielsweise analog zu denen der Formel (IIIa) erfolgen, wobei die stöchiometrischen Verhältnisse bei der Umsetzung so gewählt werden, daß die Positionen 2,2' bzw. 7,7' funktionalisiert werden (siehe z.B. J. H. Weisburger, E. K. Weisburger, F.E. Ray, J. Am. Chem. Soc. 1959, 72, 4253; F.K. Sutcliffe, H. M. Shahidi, D. Patersor., J. Soc. Dyers Colour 1978, 94, 306 und G. Haas, V. Prelog, Helv. Chim. Acta 1969, 52, 1202).

Die Herstellung von Verbindungen der Formel (IIIc) kann beispielsweise über eine Dibromierung in 2,2'-Stellung mit anschließender Diacetylierung in 7,7'-Stellung des 9,9'-Spirobi-9-silafluorens und anschließender Umsetzung analog zu der der Verbindungen (IIIa) erfolgen.

Verbindungen der Formel (IIIe)-(IIIg) sind beispielsweise herstellbar durch Wahl geeignet substituierter Ausgangsverbindungen beim Aufbau des Spirosilabifluorens entsprechend den beiden folgenden Reaktionsschemata:

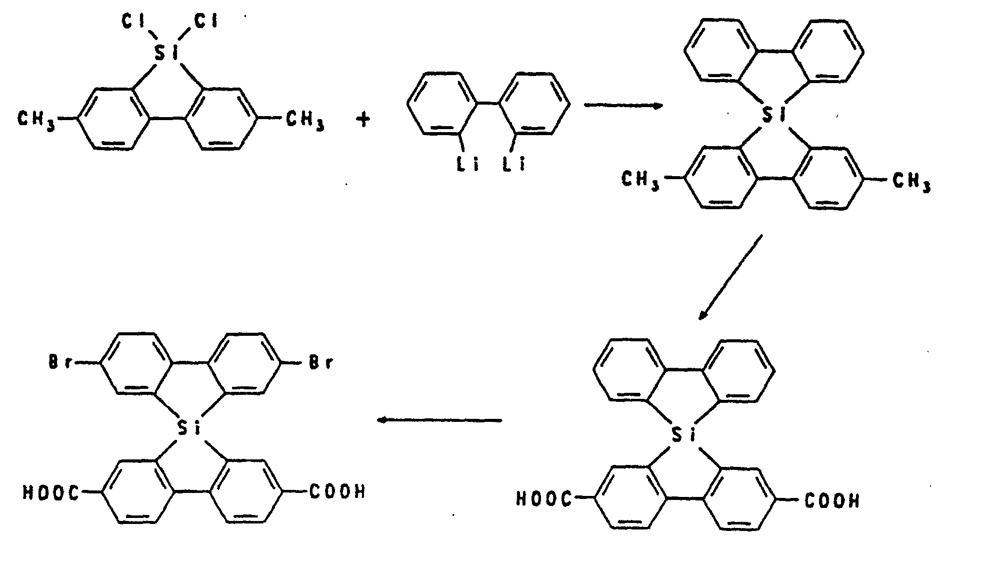

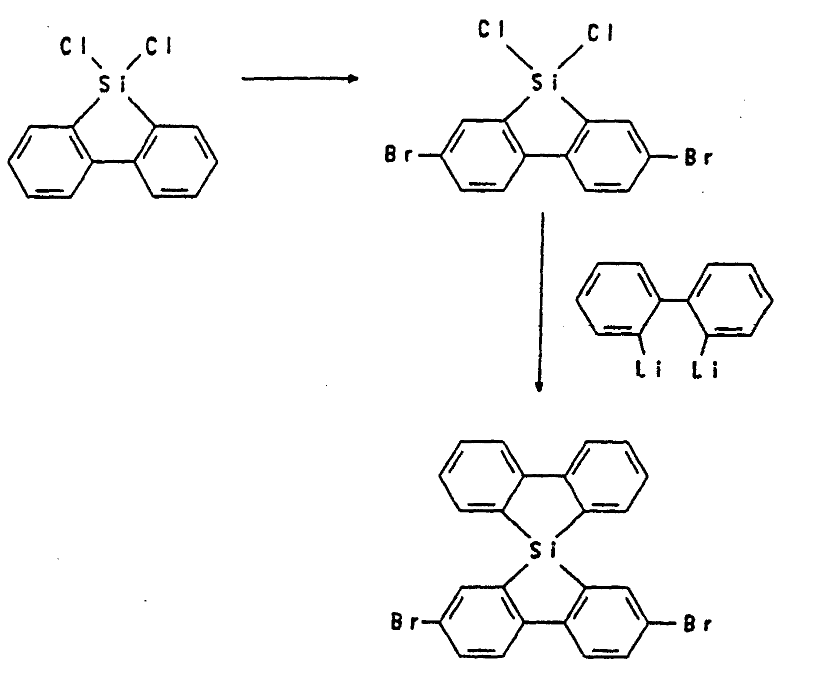

Darüber hinaus sind die dem Fachmann geläufigen Synthesesequenzen wie Nitrierung, Reduzierung, Diazotierung und Sandmeyer-Reaktion einzusetzen. Für die Synthese der Gruppen K, L, M, Q sei auf die entsprechenden Carbospiroverbindungen verwiesen.

Grundsätzlich ist es auch möglich, Oligo- oder Polymere mit den entsprechenden Spiroeinheiten einzusetzen, solange die Verbindungen in der Lage sind, in die Poren des nanokristallinen Halbleiters einzudringen. Solche Verbindungen sind in der EP-A 0 707 020; WO-A 96/17 036;
DE-A 196 06 511; DE-A 196 14 971 und DE-A 196 15 128 beschrieben; auf diese Schriften wird hiermit ausdrücklich Bezug genommen, ihre Offenbarung gilt durch Zitat als Bestandteil der vorliegenden Anmeldung.

Die erfindungsgemäße photovoltarische Zelle enthält als lichtabsorbierende Schicht vorzugsweise einen Halbleiter, der vorzugsweise eine sehr große Bandlücke, besonders bevorzugt mindestens 3,0 eV, ganz besonders bevorzugt über 3,0 eV, aufweist.
Damit eignen sich vorzugsweise Metalloxyd-Halbleiter, insbesondere die Oxyde der Übergangsmetalle, sowie der Elemente der dritten Hauptgruppe und der vierten, fünften und sechsten Nebengruppe (des periodischen Systems der Elemente) von Titan, Zirkon, Hafnium, Strontium, Zink, Indium, Yttrium, Lanthan, Vanadium, Niob, Tantal, Chrom, Molybdän, Wolfram, aber auch Oxyde von Zink, Eisen, Nickel oder Silber, Perovskite wie SrTiO₃, CaTiO₃, oder Oxyde von anderen Metallen der zweiten und dritten Hauptgruppe oder Mischoxyde oder Oxydgemische dieser Metalle. Es kann aber auch jedes andere Metalloxyd mit Halbleitereigenschaften und großem Energieabstand (Bandlücke) zwischen Valenzband und Leitungband verwendet werden.
Besonders bevorzugt als Halbleitermaterial ist Titandioxid.

Der Halbleiter weist vorzugsweise einen Rauheitsfaktor von größer als 1, besonders bevorzugt von größer als 20, ganz besonders von größer als 150 auf.
Der Rauheitsfaktor ist definiert als das Verhältnis einer wirklichen/effektiven Oberfläche zur Fläche der Projektion dieser Oberfläche eines Körpers, in diesem Fall also der Oberfläche des Halbleiters.

Der Rauheitsfaktor kann z.B.durch gravimetrische Adsorptionsmethoden bestimmt werden, wie z.B. in F. Kohlrausch, Praktische Physik, Band 1, S. 397 (Stuttgart: B.G. Teubner, 1985) beschrieben wird. Im allgemeinen beträgt die Größe der Poren 5-200 nm, vorzugsweise 10-50 nm.

Ein Verfahren zum Herstellen von polykristallinen Metalloxyd-Halbleiterschichten mit dem SOL-GEL-Verfahren (beschrieben in Einzelheiten z. B. in Stalder und Augustynski, J. Electrochem. Soc. 1979, 126, 2007), wo beim Verfahrensschritt der Hydrolyse des Metall-Alkoholats die prozentuale relative Feuchtigkeit der Umgebungsatmosphäre in einem Bereich von 30 % bis 80 % liegen kann und innerhalb von ± 5 %, vorzugsweise ± 1 %, konstant gehalten wird, ergibt Metalloxyd-Halbleiterschichten, mit denen in erfindungsgemäßen photovoltaischen Zellen eine besonders hohe elektrische Ausbeute erzielt werden kann.
Die rauhe Oberfläche mit polykristalliner Struktur bietet eine um den Rauheitsfaktor größere Fläche für eine, vorzugsweise, monomolekulare Oberflächenschicht des Chromophors. Damit wird erreicht, daß das auf eine Fläche bestimmter Größe einfallende Licht mit bedeutend höherer Ausbeute in elektrische Energie gewandelt wird. Der Halbleiter kann als für den einfallenden Lichtstrom durchsichtig betrachtet werden. Licht wird aber teilweise auf der Oberfläche reflektiert und gelangt z. T. auf benachbarte Oberflächen. Das in den Halbleiter eindringende und nicht absorbierte bzw. gewandelte Licht trifft z. T. direkt und zu einem anderen Teil indirekt und zu einem weiteren Teil indirekt, nach Totalreflexion an der Oberfläche auf der Austrittsseite auf Chromophormoleküle, womit es gelingt, eine bedeutend höhere Lichtausbeute zu erzielen.

Als Beispiel für die Herstellung einer Titanoxydschicht (TiO₂) mit hohem Rauheitsfaktor auf einem Titansubstrat, wird nachfolgend das SOL-GEL-Verfahren beispielhaft beschrieben.
Das Titansubstrat aus reinem Titanium von etwa 99,5 % Reinheit wird zuerst während etwa 30 Minuten in etwa 18 %iger kochender HCI gereinigt. Die Titan-Ethoxyd-Lösung kann z. B. durch die Lösung von 21 mMol TiCl₄ in 10 ml sehr reinem Ethanol (puriss.) erhalten werden. Diese Lösung wird dann mit sehr reinem Methanol (puriss.) verdünnt, um eine Titankonzentration im Bereich von etwa 25 bis 50 mg/ml zu erhalten. Auf das Titansubstrat gibt man einen Tropfen der Lösung und das Titan-Alkoxyd wird bei Raumtemperatur während ca. 30 Minuten bei einer Feuchtigkeit von 48 ± 1 % hydrolisiert. Danach wird das Substrat mit der hydrolysierten Schicht während ca. 15 Minuten auf ca. 450°C erhitzt. Dieser Prozeß wird mehrmals wiederholt. Nach 10- bis 15-maliger Wiederholung hat die TiO₂-Schicht eine Dicke von etwa 20 µm erreicht.
Danach wird das Substrat mit der Schicht bei etwa 500°C während etwa 30 Minuten in einer Rein-Argon-Atmosphäre (z. B. 99,997 %) ausgeheizt. Die so hergestellte TiO₂-Schicht hat einen Rauheitsfaktor im Bereich von 200. Derartige Metalloxyd-Halbleiter-Schichten (auch anderer Metalle) können nach analogen Verfahren auf anderen Substraten erzeugt werden. Die oberen Schichten des Halbleiters können gegebenenfalls, wie z. B. in der WO-A 91/16719 beschrieben, mit einem divalenten oder trivalenten Metall dotiert sein.

Die Empfindlichkeit, d. h. die photoelektronische Ausbeute für sichtbares Licht, also auch für Sonnenlicht, kann erhöht werden, indem auf der Oberfläche des Halbleiters sog. Chromophore, auch Sensibilatoren oder Dyes genannt, als Ladungsträger chemisch an- oder eingelagert (chemisorbiert) werden. Die beiden Funktionen der Lichtabsorption und der Ladungsträger-Trennung sind bei diesen photoelektronischen Systemen getrennt. Die Lichtabsorption wird vom Chromophor im Oberflächenbereich übernommen, und die Trennung der Ladungsträger erfolgt an der Grenzschicht Halbleiter/Chromophor.
Verschiedene Chromophore haben unterschiedliche spektrale Empfindlichkeiten.
Die Wahl des Chromophors kann somit der spektralen Zusammensetzung des Lichts der Lichtquelle angepaßt werden, um die Ausbeute möglichst zu vergrößern. Als Chromophore, d. h. Sensibilisatoren, eigenen sich insbesondere die Komplexe von Übergangsmetallen vom Typ Metall (L₃), Metall (L₂) von Ruthenium und Osmium (z. B. Ruthenium tris (2,2 bipyridyl-4,4'dicarboxylate), Ruthenium cis diaqua bipyridyl Komplexe, wie Ruthenium cis-diaqua bis(2,2 bipyridyl-4,4'dicarboxylate) sowie Porphyrine (z. B. Zink tetra (4-carboxyphenyl) Porphyrin) und Cyanide (z. B. Eisen-Hexacyanid-Komplexe) und Phthalocyanine.
Die Chromophore können im Bereich der Oberfläche des Metalloxyd-Halbleiters chemisorbiert, adsorbiert oder sonstwie fest angelagert sein. Günstige Resultate wurden beispielsweise mit Chromophoren erzielt, die mit Carbonsäure- oder Phosphonsäure-Liganden an die Oberfläche des Metalloxyd-Halbleiters gebunden sind.

Geeignete Chromophore sind beispielsweise auch in Chem. Rev. 1995, 49-68 beschrieben.

Besonders bevorzugt sind die Chromophore (VIII) und (IX), 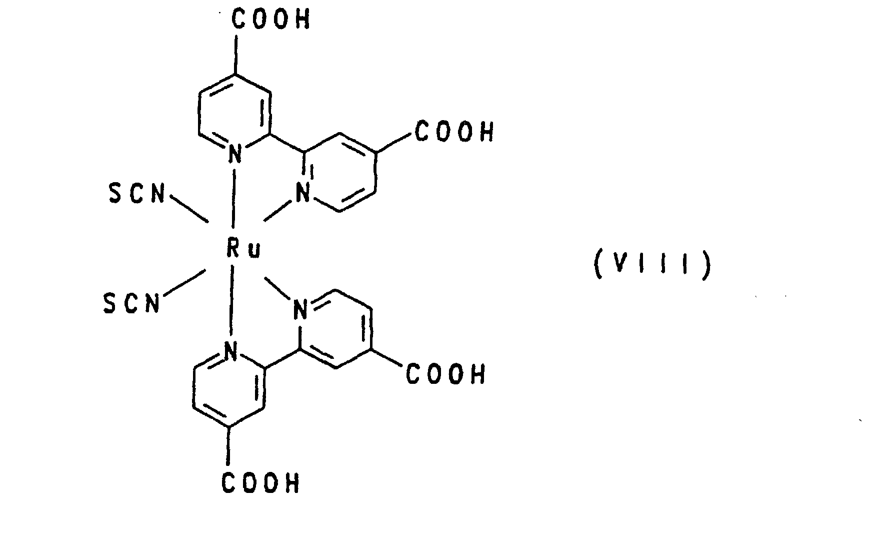 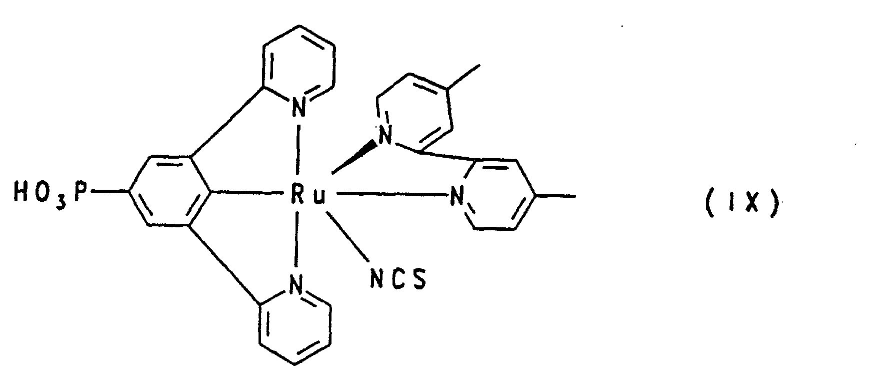 deren Synthese und Eigenschaften in J. Chem. Soc. Chem. Comm. 1995, 65 beschrieben sind.

Das Aufbringen des Chromophors, beispielsweise RuL₃⁴⁻, erfolgt z. B. durch Eintauchen des Substrats mit der Oxydschicht in eine ethanolische Lösung von 2 x 10⁻⁴M RuL₃⁴⁻, während etwa einer Stunde. Andere Chromophore lassen sich nach analogen Verfahren auf Titanoxyd oder andere Metalloxyd-Halbleiter aufbringen.

Als Elektrode 15 eignen sich stabile, metallisch leitende Substanzen, z. B. Au, Ag, Pt, Cu, oder andere Metalle. Es können aber auch, für einige Anwendungen vorzugsweise lichtdurchlässige leitfähige Substanzen, wie dotierte Metalloxide, z. B. Indium-Zinn-Oxid, Sb-dotiertes Zinnoxid oder Al-dotiertes Zinkoxid, verwendet werden. Dabei kann die Austrittsarbeit des verwendeten Elektrodenmaterials vorzugsweise an das lonisationspotential des verwendeten Lochtransportmaterials angepaßt werden.

Die Elektrode kann, wie in der EP-A 0 333 641 beschrieben, auf ein transparentes Substat, z. B. Glas aufgebracht werden, und mit der Lochtransportschicht verbunden sein. Vorzugsweise kann sie in der in dieser Erfindung beschriebenen Zelle durch physikalische Abscheidemethoden, z. B. Aufdampfen oder Zerstäubern (Sputtern) direkt auf die Lochtransportschicht aufgebracht werden, ohne daß eine zweite Glasplatte notwendig ist. Dieses Verfahren ist in Anwendungen zu bevorzugen, wenn das Gewicht der Zelle vermindert werden soll.

Gegebenenfalls kann die Elektrode auch, wie in der WO-A 93/19479 beschrieben, mit einem weiteren Halbleiter beschichtet sein. Als elektrisch isolierende Materialien 16 und 17 bzw. gegebenenfalls als seitlicher Rahmen für die erfindungsgemäße Zelle eignen sich beispielsweise Kunststoff oder Glas.

Gegenstand der Erfindung ist daher auch ein Verfahren zur Herstellung einer photovoltaischen Zelle, dadurch gekennzeichnet, daß man auf einem leitfähigen festen Träger
a) ein Halbleiterkolloid aufbringt,
b) darauf gegebenenfalls einen Farbstoff aufbringt,
c) darauf eine Transportschicht, enthaltend ein Lochleitermaterial, aufbringt,
d) darauf die Gegenelektrode aufbringt, und auf diese
e) eine isolierende Schicht aufbringt.

Falls gewünscht kann die Zelle z. B. mit einem Kleber oder einer Folie versiegelt werden.

Die erfindungsgemäße photovoltaische Zelle hat im allgemeinen eine Dicke von 5 bis 20 mm (mit Substrat).
Sie kann zur Vermeidung von Reflexionsverlusten mit einer ein-, zwei- oder mehrlagigen Antireflexschicht versehen sein.

Zur Erhöhung der Lichtausbeute kann die Rückseite der Zelle so konstruiert sein, daß Licht diffus in die Zelle zurückreflektiert wird.

Eine weiter Erhöhung der Lichtausbeute kann beispielsweise dadurch erzielt werden, daß das einfallende Sonnenlicht beispielsweise durch Spiegel oder Fresnellinsen konzentriert wird.

Die erfindungsgemäße Zelle kann auch Teil einer Tandemzelle sein; bei solchen Vorrichtungen wandeln mehrere Teilzellen Licht aus unterschiedlichen Spektralbereichen in elektrische Energie um.

Die erfindungsgemäße Zelle wird als Photozelle verwendet, d. h., sie dient zum Erzeugen von elektrischer Energie aus Licht. Vorzugsweise handelt es sich um eine Solarzelle, d. h. eine Vorrichtung zum Erzeugen elektrischer Energie aus Sonnenlicht.

Auf den Inhalt der deutschen Patentanmeldung 195 33 850.2, deren Priorität die vorliegende Anmeldung beansprucht, wird hiermit ausdrücklich Bezug genommen, sie gilt, wie auch die Zusammenfassung der vorliegenden Anmeldung, durch Zitat als Bestandteil der Beschreibung.

Die Erfindung wird durch die Beispiele näher erläutert.

### Synthesebeispiele

### Beispiel 1

### 9,9 '-Spirobifluoren

7,66 g Magnesiumspäne und 50 mg Anthracen wurden in 100 ml trockenem Diethylether in einem 11 Dreihalskoben mit Rückflußkühler unter Argon vorgelegt und mit 75 g 2-Brombiphenyl gelöst in 60 ml trockenem Diethylether umgesetzt. Anschließend wurden 56,77 g 9-Fluorenon gelöst in 500 ml trockenem Diethylether unter Rühren zugetropft. Nach beendeter Zugabe wurde 2 Stunden weiter gekocht. Der ausgefallene gelbe Magnesiumkomplex wurde abgesaugt und mit Ether gewaschen. Der abfiltrierte gelbe Magnesiumkomplex wurde dann in einer Lösung aus 48 g Ammoniumchlorid in 800 ml Eiswasser hydrolysiert .Nach 60 min. Rühren wurde das gebildete Fluorenol abgesaugt, mit Wasser gewaschen, und trockengesaugt Das getrocknete Produkt wurde dann in ca. 800 ml Eisessig unter Zugabe von 3 ml HCI conc. 2 h am Rückfluß gekocht. Nach Abkühlung wurde das Produkt abgesaugt, mit Wasser gewaschen und getrocknet. Zur weiteren Reinigung wurde einmal aus Aceton umkristallisiert. Man erhielt 86 g 9,9'-Spirobifluoren als farblose Kristalle (82 % Ausbeute).

### Beispiel 2

### Synthese von 2,2',4,4',7,7'-Hexabromo-9,9'-spirobifluoren

Zu einer Lösung von 3,16 g (10 mmol) 9,9'-Spirobifluoren in 20 ml Methylenchlorid wurden 200 mg wasserfreies FeCl₃ gegeben und mit Ultraschall behandelt. Der Reaktionskolben wurde mit Al-Folie vor Lichtzutritt geschützt. Anschließend wurden in der Siedehitze 9,85 g (3.15 ml, 62 mmol) Brom in 5 ml Methylenchlorid innerhalb von 15 min zugetropft. Die Lösung wurde weitere 20 h am Rückfluß gekocht und mit Ultraschall behandelt. Nach Abkühlung wurde Petrolether versetzt und abgesaugt. Zur weiteren Reinigung wurde aus THF / Methanol umkristallisiert und 5 h bei 80°C getrocknet. Ausbeute 6,15 g (77 %) farblose Kristalle.

### Beispiel 3

### Synthese von 2,2',4.4',7,7 '-Hexabiphenylyl-9,9'-spirobifluoren (Verbindung 1)

In einem 250 ml Zweihalskolben mit Rückflußkühler, KPG-Rührer wurden 1,6 g Hexabromspirobifluoren und 3 g Biphenylboronsäure in einer Mischung aus 50 ml Toluol und 50 ml 1 M Kaliumcarbonatlösung aufgeschlämmt. Die Mischung wurde unter Stickstoff zum Rückfluß erhitzt und 115 mg Tetrakis-(triphenylphosphin)palladium in 5 ml Toluol zugegeben. Anschließend wurde unter Rühren weitere 7 h am Rückfluß gekocht. Nach Beendigung der Reaktion wurde die abgekühlte Lösung abfiltriert und das Filtrat 2 x mit Wasser ausgeschüttelt (zur besseren Phasentrennung wurde Chloroform zugesetzt). Die organische Phase wurde über Natriumsulfat getrocknet, über eine kurze Säule mit Kieselgel filtriert und anschließend das Lösungsmittel am Rotationsdampfer abgezogen. Zur weiteren Reinigung wurde aus Dichlormethan / Pentan umkristallisiert. Man erhielt 2 g (80 %) farblose, unter UV-Beleuchtung blau fluoreszierende Kristalle.

### Beispiel 4

### 2,2',7,7 '-Tetrajod-9,9 '-spirobifluoren

In einem 100 ml Zweihalskolben mit Rückflußkühler und Trockenrohr wurden 3,16 g (10 mmol) 9,9'-Spirobifluoren, gelöst in 30 ml Chloroform, bei Raumtemperatur mit 5,8 g (22,8 mmol) lod versetzt und anschließend 10,75g (25 mmol) Bis-(trifluoracetoxy)-iodbenzol zugegeben. Das Reaktionsgemisch erwärmte sich auf ca 40°, unter Bildung eines hellen Niederschlags. Nach 1,5 h wurde das bereits ausgefallene Produkt abgesaugt, und mit Chloroform nachgewaschen und getrocknet. Die Chloroformlösungen werden vereinigt, und nacheinander mit gesättigter Natriumsulfitlösung, gesättigter Natriumcarbonatlösung und Wasser gewaschen. Nach dem Trocknen über Natriumsulfat wurde eingeengt und eine zweite Produktfraktion erhalten. Beide Produktfraktionen wurden vereinigt, in Aceton aufgekocht und nach Abkühlung abgesaugt. Man erhielt 2,2',7,7'-Tetrajod-9,9'-spirobifluoren als feinkristallines farbloses Pulver mit 8,1 g in nahezu quantitativer Ausbeute.
¹H-NMR (CDCI3, ppm): 6.98 (d, J = 1,48 Hz, 4 H, H-1,1',8,8'); 7.54 (dd, J = 7.88, 1.48 Hz, 4 H, H-3,3',6,6'); 7.72 (d, J = 7.88 Hz, 4 H, H-4,4',5,5').

### Beispiel 5

### 2,2',7,7'-Tetrakis(diphenylamino)-9,9'-spirobifluoren (Verbindung 2)

2,1 g (2,56 mmol) Tetraiodspirobifluoren wurden mit 2,25 g (13,3 mmol) Diphenylamin unter Zusatz von 2,76 g (20 mmol) Kaliumcarbonat, 635 mg (10 mmol) Kupferpulver und 208 mg (0,79 mmol) 18 - Krone-6 in 10 ml o-Dichlorbenzol unter Stickstoff 48 h zum Sieden erhitzt. Nach Abkühlung wurden die anorganischen Bestandteile abfiltriert und mit warmem Dichlorbenzol nachgewaschen. Das Dichlorbenzol wurde durch Vakuumdestillation (100 mbar, 135 - 140°C) entfernt. Der Rückstand wurde in Chloroform gelöst, über eine kurze Kieselgelsäule abfiltriert und eingeengt. Zur Reinigung wurde erst aus Chloroform/Aceton und anschließend aus Chloroform/Diethylether unter Zusatz von 2 Tropfen Hydrazinhydrat umkristallisiert. Man isolierte mit 56% Ausbeute 1,4 g 2,2',7,7'-Tetrakis-(diphenylamin)-9,9'-spirobifluoren in Form sehr feiner, gelblich-weißer Kristallnadeln.
¹H-NMR (CDCl₃, ppm): 6.69 (d, J = 1,83 Hz, 4 H, H-1,1 ',8,8'); 6.92 (dd, J = 8.18, 1.99 Hz, 4 H, H-3.3',6,6'); 6.98 (m, 24 H}; 7.20 (m, 16 H); 7.45 (d, J = 8.18 Hz, 4 H, H-4,4',5,5').

### Beispiel 6

### N,N,N',N',N'',N'',N''',N'''-Octakis(4-methoxyphenyl)-9,9'-Spirobifluoren-2,2',7,7'-tetramin

Die zur obigen Vorschrift analoge Umsetzung von Tetraiodspirobifluoren mit 4,4'-Dimethoxydiphenylamin lieferte mit vergleichbarer Ausbeute N,N,N',N',N",N",N"',N"'-Octakis(4-methoxyphenyl)-9,9'-Spirobiftuoren-2,2',7,7'-tetramin als gelblich gefärbtes Kristallpulver.
¹H-NMR (CDCl₃, ppm): 3.76 (s, 24 H, OCH₃); 6.54 (d, J = 1,99 Hz, 4 H, H-1,1',8,8'); 6.75 (dm, J = 9.07 Hz,16 H); 6.79 (dd, J = 8.18, 1.99 Hz, 4 H, H-3,3',6,6'); 6.90 (dm, J = 9.07 Hz, 16 H); 7.35 (d, J = 8.18 Hz, 4 H, H-4,4',5,5').

### Anwendungsbeispiele

### Beispiel 7

Die in Beispiel 3 beschriebene Verbindung 1 wurde bei einer Konzentration von 50 g/l in Tetrahydrofuran gelöst. Durch Eintauchen in die Lösung wurde ein Substrat beschichtet, das aus leitfähigem, SnO₂-beschichtetem Glas bestand, auf dem auf einer Seite eine glatte Schicht aus Nb-dotiertem Titandioxid aufgebracht worden war (Substrat I). Durch ein Tauchverfahren wurden beide Seiten des Substrats beschichtet. Auf der mit Titandioxid beschichteten Seite wurde anschließend durch thermisches Aufdampfen eine dünne Schicht aus Gold abgeschieden. Die mit Titandioxid und Gold beschichtete Seite wird im folgenden als die aktive Seite, die andere als die inaktive Seite bezeichnet.

Die so präparierte Probe wurde in einem optischen Aufbau montiert, der aus einer Hochdrucklampe, optischen Filtern, Linsen und Halterungen bestand. Durch den Einsatz von Filtern und das Verschieben der Linsen konnte die Intensität variiert werden. Ferner wurde das Licht mit Wellenlänge unterhalb von 380 nm im wesentlichen ausgefiltert, um eine direkte Anregung von Verbindung 1 durch Licht zu verhindern. Die Probe wurde mit der inaktiven Seite zur Lampe gerichtet montiert, so daß das Restlicht im Bereich des Absorptionsspektrums von Verbindung 1 von der sich auf der inaktiven Seite befindenden Schicht absorbiert wurde. Durch die Dotierung mit Nb wies die Titandioxidschicht eine geringe Absorption zwischen 400 und 450 nm auf, so daß diese von der Lampe angeregt wurde.

Die Gold- und SnO₂-Schichten wurden kontaktiert und an ein Strommeßgerät angeschlossen, während die Probe belichtet wurde. Es wurde keine externe Spannung angelegt. Während der Belichtung der Probe wurde ein Strom beobachtet, der nach Abblenden der Lichtquelle wieder verschwand. Bei Vergleich mit einer thermischen Behandlung der Probe zeigte, daß der beobachtete Strom ein echter Photostrom war, der durch Injektion von positiven Ladungsträgern (Löchern) in die Schicht aus Verbindung 1 und Transport der Löcher durch diese Schicht entsteht. Die Intensität der Belichtung wurde über einen Faktor zehn variiert; über diesen Bereich wuchs der Photostrom linear mit der Intensität.

### Beispiel 8

Die Verbindung 1 wurde bei einer Konzentration von 50 g/l in Tetrahydrofuran gelöst. Durch Eintauchen in die Lösung wurde ein Substrat beschichtet, das aus leitfärugem, SnO₂-beschichtetem Glas bestand, auf dem auf der einen Seite eine nanoporöse Schicht aus Titandioxid aufgebracht worden war, wobei der Anteil der oberhalb 400 nm noch schwach absorbierenden Rutilphase etwa 30 % betrug und die Oberfläche einen Rauheitsfaktor von 700-1000 aufwies (Substrat II). Durch ein Tauchverfahren wurden beide Seiten des Substrats beschichtet. Auf der mit Titandioxid beschichteten Seite wurde anschließend durch thermisches Aufdampfen eine dünne Schicht aus Gold aufgebracht. Die mit Titandioxid und Gold beschichtete Seite wird im folgenden als die aktive Seite, die andere als die inaktive Seite bezeichnet.

Die so präparierte Probe wurde in dem in Beispiel 7 beschriebenen optischen Aufbau montiert. Die Probe wurde mit der inaktiven Seite zur Lampe gerichtet montiert, so daß das Restlicht im Bereich des Absorptionsspektrums von Verbindung 1 von der sich auf der inaktiven Seite befindenen Schicht absorbiert wurde. Durch den Anteil an der Rutilphase wies die Titandioxidschicht eine geringe Absorption zwischen 400 und 430 nm auf, so daß diese von der Lampe angeregt wurde.

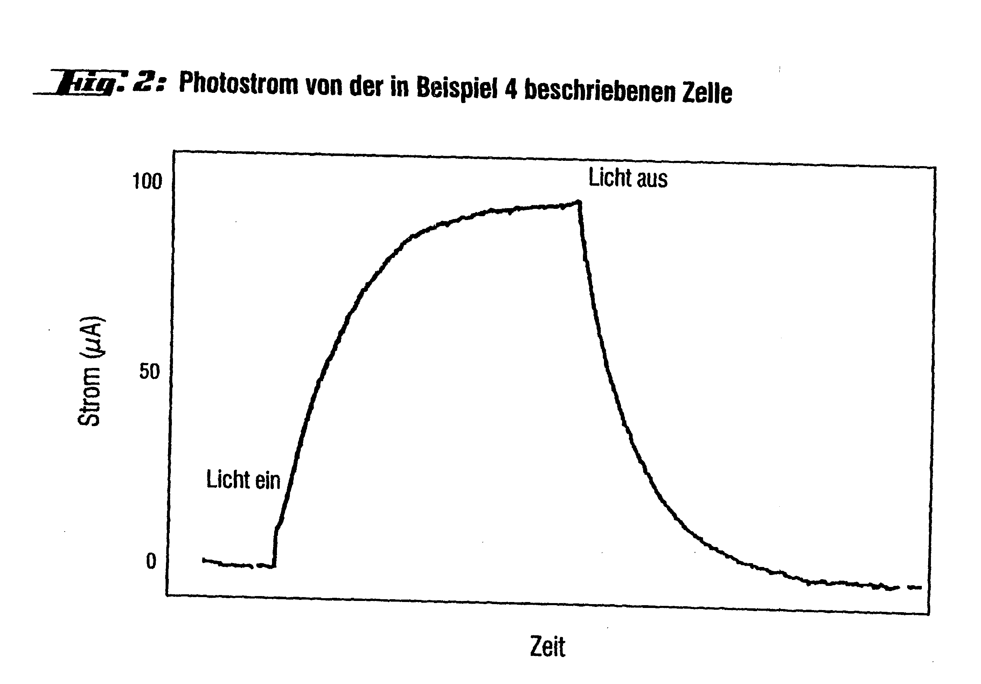

Die Gold- und SnO₂-Schichten wurden kontaktiert und an einem Strommeßgerät angeschlossen, während die Probe belichtet wurde. Es wurde keine externe Spanung angelegt. Während der Belichtung der Probe wurde ein Strom beobachtet, der nach Abblenden der Lichtquelle wieder verschwand. Bei Vergleich der thermischen Behandlung der Probe zeigte sich, daß der beobachtete Strom ein echter Photostrom war, der durch Injektion von positiven Ladungsträgern (Löchern) in die Schicht aus Verbindung 1 und Transport der Löcher durch diese Schicht entsteht. Die Intensität der Belichtung wurde über einen Faktor zehn variiert; über diesen Bereich wuchs der Photostrom linear mit der Intensität. Der Photostrom war um ein Vielfaches höher als der Photostrom der im Beispiel 7 beschriebenen Probe, was darauf hinweist, daß Verbindung 1 in die Poren der Schicht eindringt (siehe Fig. 2).

### Beispiel 9

Die in Beispiel 7 beschriebene Probe wurde in einem optischen Aufbau montiert, der aus einem gepulsten, durchstimmbaren Laser, einer Weißlichtquelle, einem Monochromator und Abbildungs- und Detektionsoptik bestand. Der Laser lieferte bei 30 Hz Pulse mit einer Dauer von ca. 2 ns bei 420 nm, d.h. außerhalb der Absorption von Verbindung 1, aber noch innerhalb der Absorption der Nb-dotierten Titandioxidschicht. Während die Probe so bestrahlt wurde, wurde die Transientenabsorption des Radikalkations von Verbindung 1 bei 500 nm beobachtet. Der Anstieg der Absorption fand innerhalb der Zeitauflösung des Experiments statt. Dies weist auf eine sehr effektive und schnelle Injektion der Ladungsträger in die Schicht aus Verbindung 1 hin.

### Beispiel 10

Auf einen SnO₂ -beschichteten Glasträger wurde eine nanoporöse Schicht aus TiO₂ durch Siebdruck mit einer nach der Sol-Gel-Methode hergestellten Suspension aufgetragen und in einem heißen Luftstrom bei ca. 400 °C für ca. 20 Minuten getrocknet. Die Schichtdicke beträgt ca 1.5 µm, die Schicht besteht fast vollkommen aus der Anatase-Phase und weist damit oberhalb 400 nm keine Absorption auf. Der beschichtete Träger wurde bei einer Temperatur von ca. 50 °C in eine 10⁻⁴ M ethanolische Lösung von Ruthenium tris (2,2'bipyridyl-4,4'-dicarboxylat) eintegaucht. Nach ca. 2 Stunden wurde der Träger aus der Lösung herausgenommen, mit Ethanol gespült und kurz im warmen Luftstrom getrocknet.
Das Schichtsystem wies eine maximale Absorbanz von ca. 0,2 bei ca. 500 nm auf. Die Fläche der Probe betrug ca. 0,3 cm².

In 5 ml Chloroform wurden 100 mg der Verbindung 2 gelöst. Die Lösung wurde durch Eintauchen von Kapillarröhrchen in die Lösung und Tupfen auf die Farbstoffoberfläche in die Poren der Schicht eingesogen. Danach wurde ein Tropfen der Lösung direkt auf die Oberfläche gegeben und bei Raumtemperatur getrocknet. Danach wurde der beschichtete Träger in eine Aufdampfapparatur montiert, in der bei einem Vakuum von ca. 10⁻⁵ mbar eine weitere 100 nm dicke Schicht aus Verbindung 2 mittels thermischer Verdampfung aufgetragen wurde. Der beschichtete Träger wurde weiterhin in einer Aufdampfapparatur mit einer 200 nm dicken Goldschicht als Gegenelektrode beschichtet.

Die so präparierte Probe wurde in dem in Beispiel 7 beschriebenen Aufbau montiert. Für die Messungen wurde durch Einsatz von entsprechenden optischen Filtern das Licht mit einer Wellenlänge unterhalb von 430 nm blockiert. Ferner wurde der Aufbau so justiert, daß die Intensität der Strahlung in etwa der Intensität von Sonnenlicht in Mitteleuropa entsprach (ca. 750 W/m²).

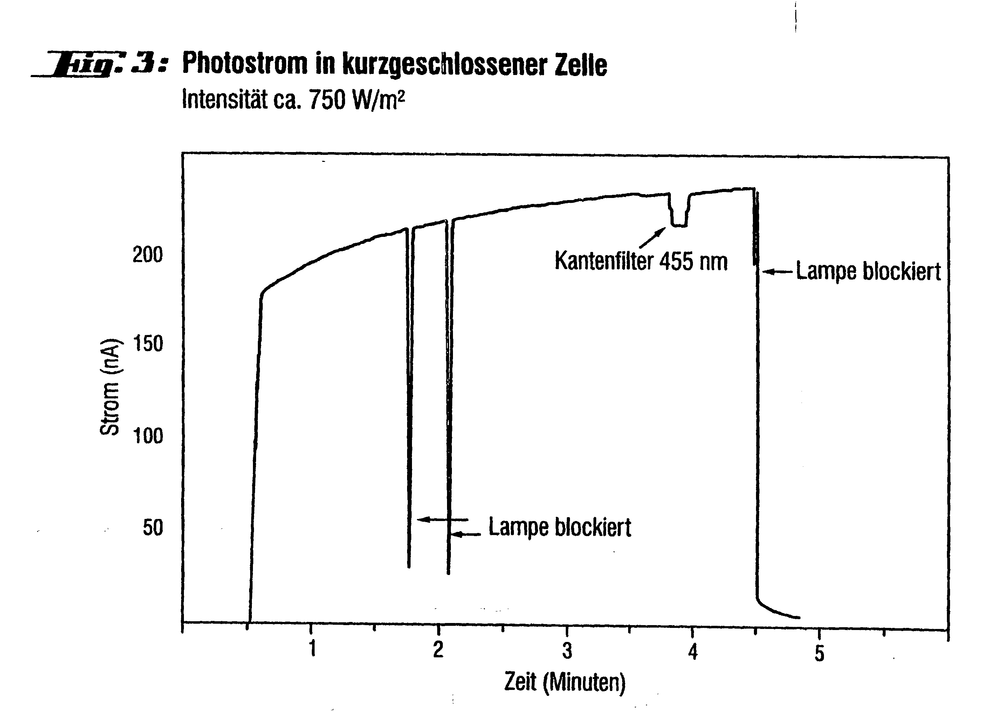

Die Gold und SnO₂ Schichten wurden kontaktiert und an einem Potentiostaten angeschlossen, während die Probe belichtet wurde. Ohne externe Spannung wies die Probe unter Belichtung einen Strom von ca. 200 nA auf, ohne Belichtung aber keinerlei Strom. Wenn ein Kantenfilter bei 470 nm eingebaut wurde, entsprach die Photostromabnahme in etwa der Abnahme der Lichtabsorption vom Farbstoff (siehe Fig. 3).

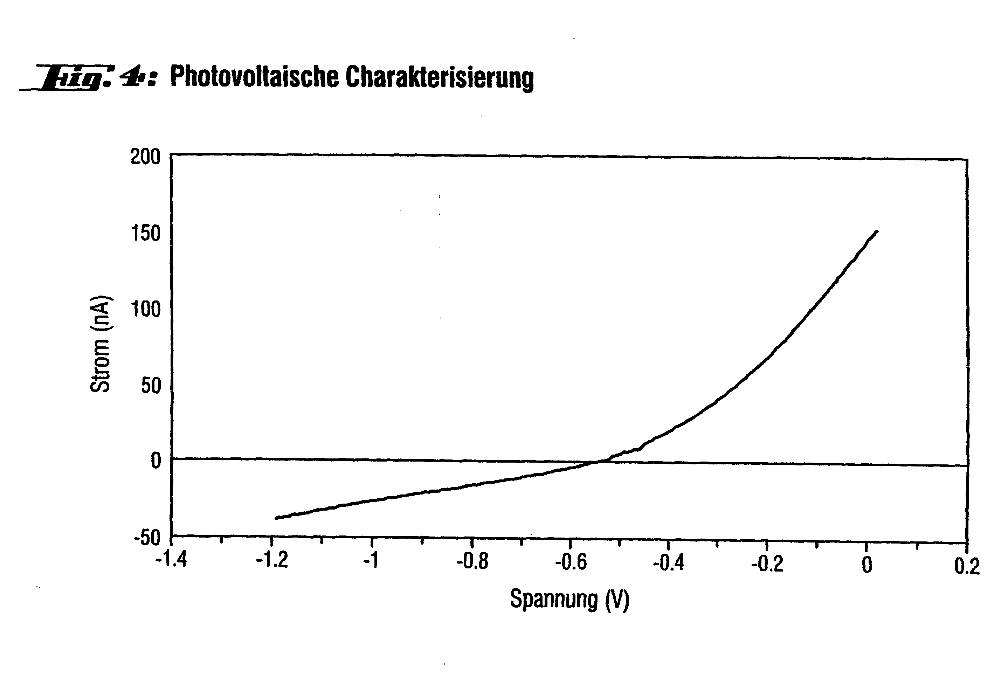

Die Strom-Spannungs-Charakteristika der Probe wurden mit und ohne Belichtung gemessen. Ohne Belichtung floß kein meßbarere Strom, selbst bei angelegter externer Spannung. Unter Belichtung wurden die Charakteristika einer photovoltaischen Zelle mit einer Leerlaufspannung von ca. 500-600 mV und einem Kurzschlußsstrom von ca. 800 nA/cm² gemessen (siehe Fig. 4).

### Beispiel 11

Auf einen SnO₂ -beschichteten Glasträger wurde eine nanoporöse Schicht aus TiO₂ durch Siebdruck mit einer nach der Sol-Gel-Methode hergestellten Suspension aufgetragen und in einem heißen Luftstrom bei ca. 400 °C für ca. 20 Minuten getrocknet. Die Schichtdicke beträgt ca 1.5 µm, die Schicht besteht fast vollkommen aus der Anatase-Phase und weist damit oberhalb 400 nm keine Absorption auf. Der beschichtete Träger wurde bei einer Temperatur von ca. 50 °C in eine 10⁻⁴ M ethanolische Lösung von Ruthenium tris (2,2'bipyridyl-4,4'-dicarboxylat) eintegaucht. Nach ca. 2 Stunden wurde der Träger aus der Lösung herausgenommen, mit Ethanol gespült und kurz im warmen Luftstrom getrocknet.
Das Schichtsystem wies eine maximale Absorbanz von ca. 0,2 bei ca. 500 nm auf. Die Fläche der Probe betrug ca. 0,3 cm².

In 5 ml Chlorobenzol wurden 100 mg der Verbindung 2 gelöst. Die Lösung wurde durch Eintauchen des Substrats in die Lösung mit einer Geschwindigkeitvon 5 cm/min in die Poren der Schicht eingesogen. Danach wurde der beschichtete Träger in eine Aufdampfapparatur montiert, in der bei einem Vakuum von ca. 10⁻⁵ mbar eine weitere 100 nm dicke Schicht aus Verbindung 2 mittels thermischer Verdampfung aufgetragen wurde. Der beschichtete Träger wurde weiterhin in einer Aufdampfapparatur mit einer 200 nm dicken Goldschicht als Gegenelektrode beschichtet.

Die so präparierte Probe wurde in dem in Beispiel 7 beschriebenen Aufbau montiert. Für die Messungen wurde durch Einsatz von entsprechenden optischen Filtern das Licht mit einer Wellenlänge unterhalb von 430 nm blockiert. Ferner wurde der Aufbau so justiert, daß die Intensität der Strahlung in etwa der Intensität von Sonnenlicht in Mitteleuropa entsprach (ca. 750 W/m²).

Die Gold und SnO₂ Schichten wurden kontaktiert und an einem Potentiostaten angeschlossen, während die Probe belichtet wurde. Ohne externe Spannung wies die Probe unter Belichtung einen Strom von ca. 7 µA auf, ohne Belichtung abe- keinerlei Strom.

Die Strom-Spannungs-Charakteristika der Probe wurden mit und ohne Belichtung gemessen. Ohne Belichtung floß kein meßbarere Strom, selbst bei angelegter externer Spannung. Unter Belichtung wurden die Charakteristika einer photovoltaischen Zelle mit einer Leerlaufspannung von ca. 500 mV und einem Kurzschlußsstrom von ca. 14 µA/cm² gemessen.

## Patentansprüche

1. Photovoltaische Zelle, **dadurch gekennzeichnet, daß** als Ladungstransportschicht eine oder mehrere Spiroverbindungen der Formel (I) als Lochleitermaterialen 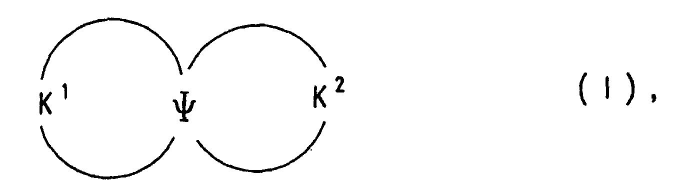 wobei
ψ C, Si, Ge oder Sn, vorzugsweise C, Si, Ge, besonders bevorzugt C, Si und insbesondere C, und
K¹ und K² unabhängig voneinander konjugierte Systeme
bedeuten, verwendet werden.

2. Photovoltaische Zelle nach Anspruch 1, **dadurch gekennzeichnet, daß** als Lochleitermaterial ein oder mehrere 9,9'-Spirobifluorenderivate der Formel (II) 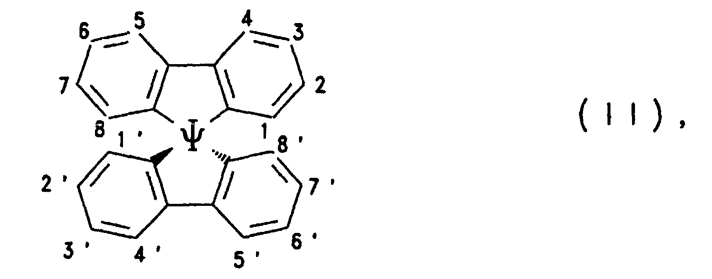 wobei ψ in Formel (I) in Anspruch 1 angegebenen Bedeutungen hat und die Benzogruppen unabhängig voneinander substituiert und/oder anelliert sein können, verwendet werden.

3. Photovoltaische Zelle nach Anspruch 2, **dadurch gekennzeichnet, daß** als Lochleitermaterial ein oder mehrere Spirobifluorenderivate der Formel (III), 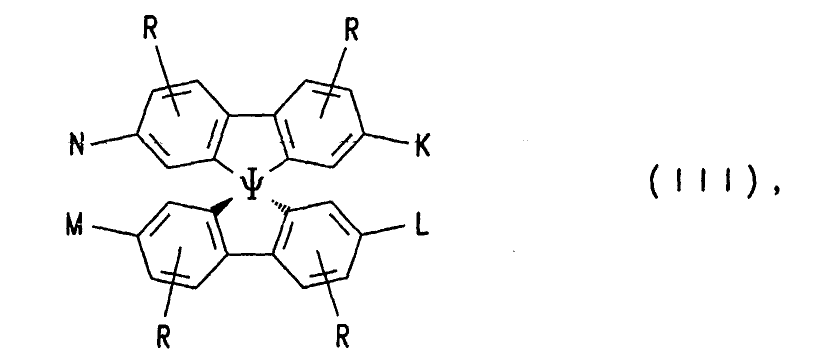 wobei die Symbole und Indizes folgende Bedeutungen haben:
ψ ist C, Si, vorzugsweise C,
K, L, M, N sind gleich oder verschieden und bedeuten eine Gruppe der Formeln 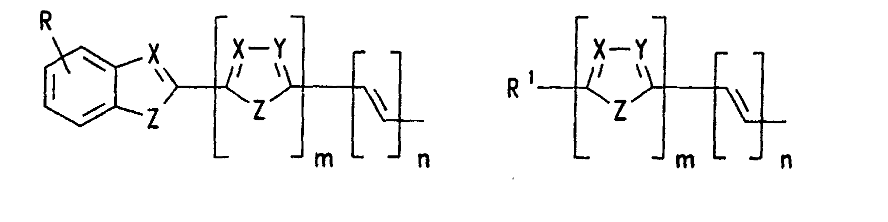 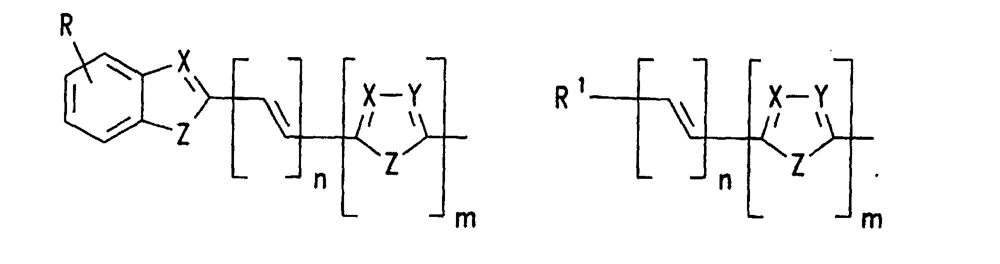 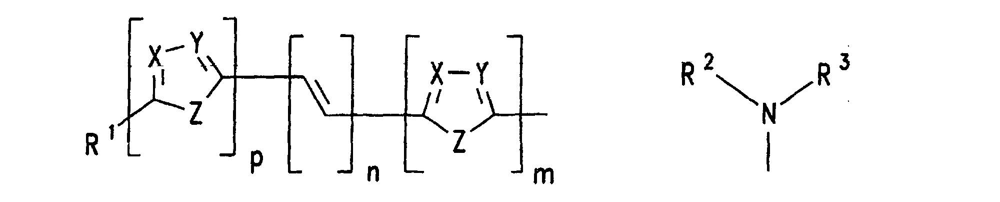
R kann, gleich oder verschieden, die gleichen Bedeutungen wie K, L, M, N haben oder ist -H, eine lineare oder verzweigte Alkyl, Alkoxy oder Estergruppe mit 1 bis 22, vorzugsweise 1 bis 15, besonders bevorzugt 1 bis 12 C-Atomen, -CN, -NR²R³, -Ar oder -O-Ar;
Ar ist Phenyl, Biphenyl, 1-Naphthyl, 2-Naphthyl, 2-Thienyl, 2-Furanyl, 9-Anthryl, wobei jede dieser Gruppen einen oder zwei Reste R tragen kann;
m, n, p sind 0, 1, 2 oder 3;
X, Y sind gleich oder verschieden CR oder Stickstoff;
Z ist -O-, -S-, -NR¹-, -CR¹R⁴-, -CH = CH-, -CH = N-;
R¹, R⁴ können, gleich oder verschieden, die gleichen Bedeutungen wie R haben;
R², R³ sind gleich oder verschieden H, eine lineare oder verzweigte Alkylgruppe mit 1 bis 22 C-Atomen, -Ar, 3-Methylphenyl,
verwendet werden.

4. Photovoltaische Zellen nach einem oder mehreren der vorhergehenden Ansprüche, **gekennzeichnet durch** eine lichtabsorbierende Schicht,die ein Halbleitermaterial mit einer Bandlücke von mindestens 3 eV enthält.

5. Photovoltaische Zelle gemäß Anspruch 4, **dadurch gekennzeichnet, daß** das Halbleitermaterial ein Metalloxid ist.

6. Photovoltaische Zelle nach Anspruch 4 oder 5, **dadurch gekennzeichnet, daß** die Halbleiteroberfläche einen Rauhigkeitsfaktor von > 20 aufweist.

7. Photovoltaische Zelle nach einem oder mehreren der vorhergehenden Ansprüche, **dadurch gekennzeichnet, daß** sie eine lichtabsorbierende Schicht mit einem Farbstoff als Sensibilisator enthält.

8. Photovoltaische Zelle nach einem oder mehreren der vorhergehenden Ansprüche, **dadurch gekennzeichnet, daß** das Lochmaterial amorph vorliegt.

9. Verfahren zur Herstellung einer photovoltaischen Zelle nach einem oder mehreren der Ansprüche 4 bis 8, **dadurch gekennzeichnet, daß** man auf einem leitfähigen festen Träger
a) ein Halbleiterkolloid aufbringt,
b) darauf gegebenenfalls einen Farbstoff aufbringt,
c) darauf eine Transportschicht, enthaltend das besagte Lochleitermaterial, aufbringt,
d) darauf eine Gegenelektrode aufbringt, und auf diese
e) eine isolierende Schicht aufbringt.

10. Verwendung von photovoltaischen Zellen nach einem oder mehreren der Ansprüche 1 bis 8 zur Erzeugung von Strom aus Sonnenlicht.

## Claims

1. A photovoltaic cell whose charge transport layer comprises one or more spiro compounds of the formula (I) 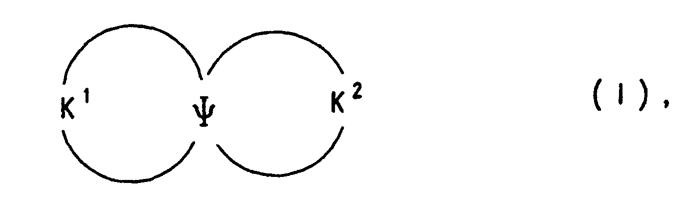 where
ψ is C, Si, Ge or Sn, preferably C, Si, Ge, particularly preferably C, Si and in particular C, and
K¹ and K², independently of one another, are conjugated systems, as hole conductor materials.

2. A photovoltaic cell as claimed in claim 1, wherein the hole conductor material used is one or more 9,9'-spirobifluorene derivatives of the formula (II) 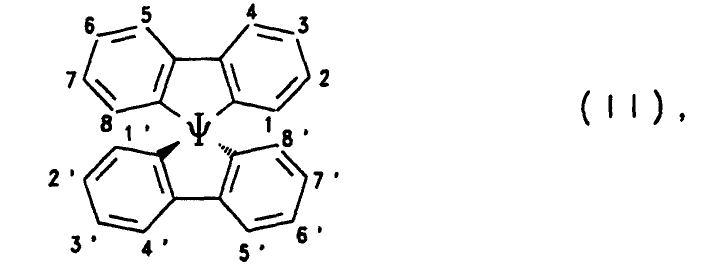 where ψ in formula (I) is as defined in claim 1 and the benzo groups can, independently of one another, be substituted and/or fused.

3. A photovoltaic cell as claimed in claim 2, wherein the hole conductor material used is one or more spirobifluorene derivatives of the formula (III), 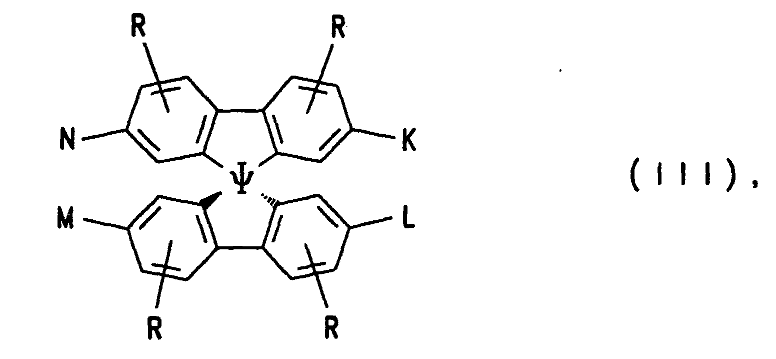 where the symbols and indices have the following meanings:
ψ is C, Si, preferably C,
K, L, M, N are identical or different and are a group of the formulae 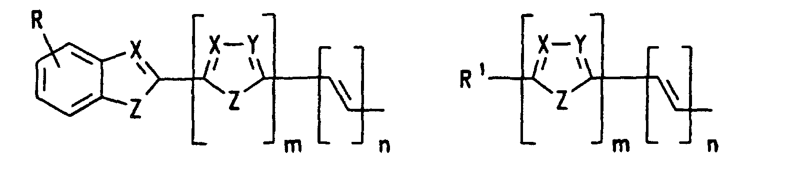 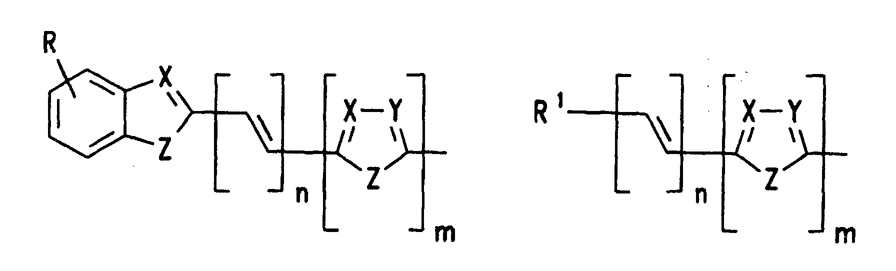 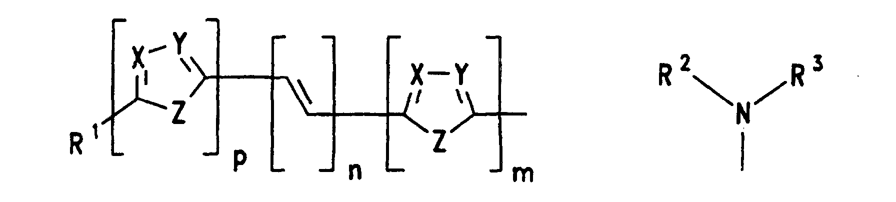
R can be identical or different and as defined for K, L, M, N or be -H, a linear or branched alkyl, alkoxy or ester group having from 1 to 22, preferably from 1 to 15, particularly preferably from 1 to 12, carbon atoms, -CN, -NR²R³, -Ar or -O-Ar;
Ar is phenyl, biphenyl, 1-naphthyl, 2-naphthyl, 2-thienyl, 2-furanyl, 9-anthryl, where each of these groups can bear one or two radicals R;
m, n, p are 0, 1, 2 or 3;
X, Y are identical or different and are CR or nitrogen;
Z is -O-, -S-, -NR¹-, -CR¹R⁴-, -CH=CH-, -CH=N-;
R¹, R⁴ can be identical or different and be as defined for R;
R², R³ are identical or different and are H, a linear or branched alkyl group having from 1 to 22 carbon atoms, -Ar, 3-methylphenyl.

4. A photovoltaic cell as claimed in one or more of the preceding claims having a light-absorbing layer comprising a semiconductor material which has a band gap of at least 3 eV.

5. A photovoltaic cell as claimed in claim 4, wherein the semiconductor material is a metal oxide.

6. A photovoltaic cell as claimed in claim 4 or 5, wherein the semiconductor surface has a roughness factor of > 20.

7. A photovoltaic cell as claimed in one or more of the preceding claims, which comprises a light-absorbing layer with a dye as sensitizer.

8. A photovoltaic cell as claimed in one or more of the preceding claims, wherein the hole conductor material is present in amorphous form.

9. A process for producing a photovoltaic cell as claimed in one or more of claims 4 to 8, which comprises
a) applying a semiconductor colloid to a conductive solid support,
b) if desired, applying a dye to the semiconductor,
c) applying a transport layer comprising the abovementioned hole conductor material to the dye,
d) applying a counterelectrode to the transport layer and
e) applying an insulating layer to the counterelectrode.

10. Use of a photovoltaic cell as claimed in one or more of claims 1 to 8 for generating electric power from sunlight.

## Revendications

1. Cellule photovoltaique, **caractérisée en ce qu'**on utilise en tant que couche de transport de charge un ou plusieurs composés spiranniques de formule (I), en tant que matériaux conducteurs de type P 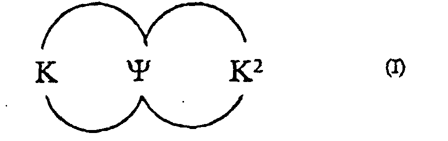 où
ψ est C, Si, Ge ou Sn, de préférence C, Si, Ge, plus particulièrement C, Si et en particulier C, et
K¹ et K² représentent indépendamment l'un de l'autre des systèmes conjugués.

2. Cellule photovoltaique selon la revendication 1, **caractérisée en ce qu'**on utilise en tant que matériau conducteur de type P un ou plusieurs dérivés de 9,9'-spirobifluorène de formule (II) 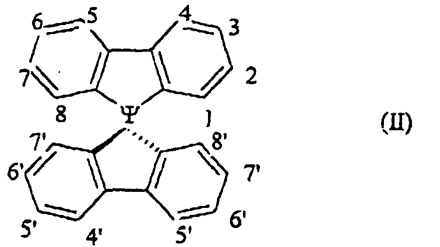 dans laquelle ψ a les significations données dans la formule (I) de la revendication 1, et les groupes benzo peuvent être substitués et/ou condensés indépendamment les uns des autres.

3. Cellule photovoltaique selon la revendication 2, **caractérisée en ce qu'**on utilise en tant que matériau conducteur de type P un ou plusieurs dérivés de spiro-bifluorène de formule (III) 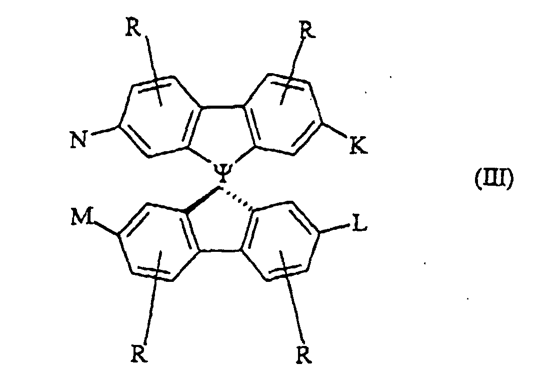 dans laquelle les symboles et indices ont les significations suivantes :
ψ est C, Si, de préférence C,
K, L, M, N sont identiques ou différents et représentent chacun un groupe ayant les formules suivantes : 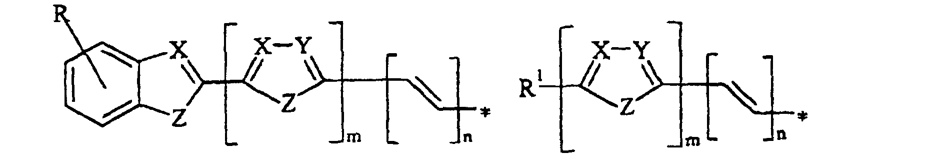 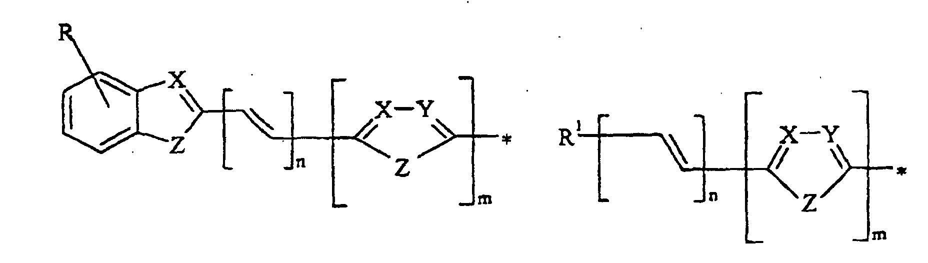 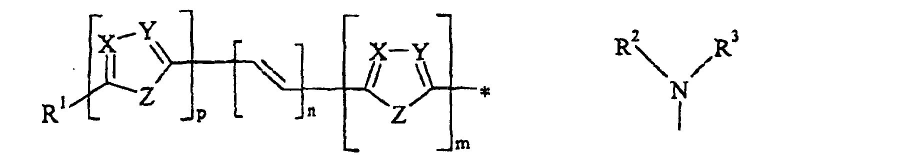
les radicaux R, identiques ou différents, peuvent avoir les mêmes significations que K, L, M, N, ou représentent chacun -H, un groupe alkyle, alcoxy ou ester à chaîne droite ou ramifiée ayant de 1 à 22, de préférence de 1 à 15 et en particulier de 1 à 12 atomes de carbone, -CN, -NR²R³, -Ar ou -O-Ar ;
Ar est un groupe phényle, biphényle, 1-naphtyle, 2-naphtyle, 2-thiényle, 2-furannyle, 9-anthryle, chacun de ces groupes pouvant porter un ou deux radicaux R ;
m, n, p valent 0, 1, 2 ou 3 ;
X, Y sont identiques ou différents et représentent chacun CR ou un atome d'azote ;
Z est -O-, -S-, -NR¹-, -CR¹R⁴-, -CH=CH-, -CH=N- ;
les radicaux R¹, R⁴, identiques ou différents, peuvent avoir les mêmes significations que R ;
les radicaux R², R³, identiques ou différents, représentent chacun H, un groupe alkyle à chaîne droite ou ramifiée ayant de 1 à 22 atomes de carbone, -Ar, le groupe 3-méthylphényle.

4. Cellule photovoltaique selon l'une ou plusieurs des revendications précédentes, **caractérisée par** une couche absorbant la lumière qui contient un matériau semi-conducteur ayant une bande interdite d'au moins 3 eV.

5. Cellule photovoltaique selon la revendication 4, **caractérisée en ce que** le matériau semi-conducteur est un oxyde métallique.

6. Cellule photovoltaique selon la revendication 4 ou 5, **caractérisée en ce que** la surface du semi-conducteur a un facteur de rugosité supérieur à 20.

7. Cellule photovoltaique selon l'une ou plusieurs des revendications précédentes, **caractérisée en ce qu'**elle contient une couche absorbant la lumière, comportant un colorant en tant que sensibilisateur.

8. Cellule photovoltaïque selon l'une ou plusieurs des revendications précédentes, **caractérisée en ce que** le matériau de type P se présente sous forme amorphe.

9. Procédé de fabrication d'une cellule photovoltaïque selon l'une ou plusieurs des revendications 4 à 8, **caractérisé en ce que**, sur un support solide conducteur
a) on applique un colloide semi-conducteur,
b) on applique par-dessus éventuellement un colorant,
c) on applique par-dessus une couche de transport contenant le matériau conducteur de type P mentionné,
d) on applique par-dessus une contre-électrode, et
e) on applique sur cette dernière une couche isolante.

10. Utilisation de cellules photovoltaïques selon l'une ou plusieurs des revendications 1 à 8 pour produire du courant à partir de la lumière solaire.
